# EUROPEAN PATENT APPLICATION

(11) **EP 1 266 937 A1**
(43) Date of publication of application: **18.12.2002**
(21) Application number: 00976293.1
(22) Date of filing: 16.11.2000
(51) Int. Cl.: C08L 101/02, C08L 65/00, C08J 5/00, C08L 45/00, C08L 33/00, C08F 8/04, C08G 8/30, C08L 61/14, G11B 7/24, C07C 69/54

(54) **THERMOPLASTIC RESIN COMPOSITION CONTAINING THERMOPLASTIC POLYMER CONTAINING ALICYCLIC GROUP AND MOLDED OBJECT**

(30) Priority: 18.11.1999 JP 32857899; 30.11.1999 JP 33971399; 11.05.2000 JP 2000138320
(71) Applicant: TEIJIN LIMITED, Osaka-shi Osaka 541-0054 (JP); BAYER AG, 51368 Leverkusen (DE)
(72) Inventor: YAMAURA, Michio, Tokio 192-0913, Japan (JP); KIDO, Nobuaki Teijin Ltd, Iwakuni Research Center, Iwakuni-shi Yamaguchi 740-0014 (JP); MATSUMURA, Shunichi Teijin Ltd, Iwakuni-shi Yamaguchi 740-0014 (JP); IWATA, Kaoru, Tokio 192-0914 (JP); HASHIDZUME, Kiyonari Teijin Limited, Iwakuni-shi Yamaguchi 740-0014 (JP); KOHNO, Kazuteru Teijin Limited, Iwakuni-shi Yamaguchi 740-0014 (JP)
(74) Representative: Hallybone, Huw George
(86) International application number: JP0008087
(87) International publication number: WO01036539

(57) **Abstract**

A thermoplastic resin composition (molding material) which suppresses dynamic brittleness caused by a reduction in molecular weight when a thermoplastic resin such as a hydrogenated styrene polymer is melt molded and does not generate crazes even when it is used at an atmosphere of high temperature and high humidity, and a substrate for optical recording media, which is made from the same.

The thermoplastic resin composition comprises a thermoplastic polymer having an alicyclic group and an addition stabilizer which can undergo addition reaction with a radical formed by the cleavage of the thermoplastic polymer.

## Description

### Field of the Invention

The present invention relates to a thermoplastic resin composition comprising a thermoplastic polymer having an alicyclic group and to molded products therefrom. More specifically, it relates to a thermoplastic resin composition which rarely experiences a reduction in molecular weight even when it is molded at a high temperature and which can provide molded products free from the generation of crazes when they are used at a high temperature and a high humidity and to molded products having the above properties therefrom.

### Prior Art

Thermoplastic polymers having a bulky alicyclic group in the main chain or side chain are synthetic resins which are excellent in terms of transparency, heat resistance, chemical resistance, solvent resistance, humidity resistance, dielectric resistance and various mechanical properties and widely used in various fields. They are non-crystalline as they have a bulky alicyclic group in the main chain or side chain, have high transparency and are used as an optical material.

The above cyclic olefin-based polymers are roughly divided into two types: one having an alicyclic group in the main chain and the other having an alicyclic group in the side chain. The polymers of the former type are further divided into addition polymers and hydrogenated ring-open polymers. As typical examples of the latter type are known hydrogenated styrene-based polymers (polyvinyl cyclohexane-based polymers).

The above addition polymers are generally obtained by addition polymerizing a cyclic olefin such as norbornene or tetracyclododecene with ethylene and/or an α-olefin. Meanwhile, the hydrogenated ring-open polymers are obtained by polymerizing a cyclic olefin such as norbornene or tetracyclododecene in the presence of a metathesis catalyst to obtain a precursor having a C=C double bond and then hydrogenating the C=C double bond. All of them have a distorted alicyclic group in the main chain and a tertiary hydrogen atom which may cause thermal cleavage in the ring. Therefore, these polymers lack thermochemical stability, namely, readily decompose thermally.

Meanwhile, the hydrogenated styrene-based polymers (polyvinyl cyclohexane-based polymers) are obtained by hydrogenating the aromatic group of the side chain of a styrene-based polymer. Although the cyclohexyl group of the side chain of the polymer is a ring having small distortion, the main chain is greatly distorted by the steric hindrance of adjacent cyclohexyl groups contained in the polymer chain. In addition, as a tertiary hydrogen atom which readily causes thermal cleavage is contained in the main chain and the cyclohexane group, respectively, it readily triggers the thermal decomposition of the polymer.

These cyclic olefin-based polymers are formed (shaped) by melt molding in most cases. Since high resin flowability is required for melt molding, it is necessary to mold at a high resin temperature. In the field which requires high transferability of fine pits, such as optical disks which are the main application field of the polymers, molding at a higher temperature is inevitable.

When the molecular weight of a polymer is reduced by the thermal decomposition of the polymer during molding under such an extreme condition, the mechanical strength of a molded product is lowered. Particularly in the case of ahydrogenated styrene-based polymer (polyvinyl cyclohexane-based polymer) whose resin temperature suitable for molding and thermal decomposition temperature are close to each other, such thermal decomposition is inevitable. A solution to the above problem is desired.

Moldability at a high temperature has been becoming more and more important along with growing capacity in optomagnetic recording disks (MO) or increasing recording density typified by the development of a digital versatile disk (DVD) and the development of a blue laser. Therefore, thermal decomposition suppression technology has been increasingly desired from cyclic olefin-based polymers which are mainly used in the above fields.

JP-A 5-242522 (the term "JP-A" as used herein means an "unexamined published Japanese patent application") discloses an optical disk substrate made from a polyvinyl cyclohexane-based resin comprising a hindered phenol-based thermal stabilizer, phosphorus-based stabilizer and a long-chain saturated fatty acid metal salt. The above hindered phenol-based thermal stabilizer and the above phosphorus-based stabilizer must have a 5 % weight reduction temperature measured by a thermal gravimeter of 295° C or more.

WO99/41307 discloses a composition comprising a hydrogenated aromatic polymer, benzofuranone and hindered phenol-based compound.

A reduction in molecular weight can be suppressed by the above stabilizers to some extent but it is hard to say that their effects are satisfactory.

For instance, when a molded product of a hydrogenated styrene-based polymer used as an optical material is used in an atmosphere of high temperature and high humidity, making use of its characteristic properties, flyspecks as large as several to more than 10 µm (called "crazes" herein according to the substance of the flyspecks) which are assumed to be caused by a decomposed product of the polymer and/or a decomposed product of the stabilizer are generated on the entire surface of the molded product. Therefore, it is difficult to use this molded product as an optical material in an atmosphere of high temperature and high humidity. Particularly when it is used as an optical disk substrate, if such a defect is slightly generated in the substrate, it greatly reduces the S/N ratio of the optical disk and its commercial value.

JP-A 59-71341 discloses a phenolic compound represented by the following formula and that this compound can be used as a synthetic resin stabilizer: 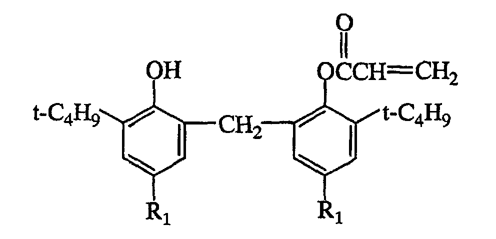 wherein R₁ is an alkyl group having 1 to 4 carbon atoms. The above publication enumerates only polyethylene, polypropylene, polystyrene, impact resistant polystyrene, ABS, polyacetal, polyamide and polyurethane as synthetic resins to be stabilized by the above stabilizer. It is also disclosed that this stabilizer has stability to heat and oxidation and heat resistant color changeability.

### Summary of the Invention

It is an object of the present invention to provide a novel molding material which suppresses a reduction in the molecular weight of a thermoplastic polymer having an alicyclic group in the main chain or side chain during melt molding.

It is another object of the present invention to provide a thermoplastic resin composition (molding material) which suppresses dynamic embrittleness caused by a reduction in the molecular weight of the above thermoplastic resin such as a hydrogenated styrene polymer during melt molding and does not generate crazes even when it is used in an atmosphere of high temperature and high humidity.

It is still another object of the present invention to provide a molded product obtained by melt molding the above molding material, particularly a substrate for optical recording media.

It is a further object of the present invention to provide a substrate for optical recording media, which rarely experiences a reduction in S/N ratio even when it is used at a high temperature and a high humidity.

Other objects and advantages of the present invention will become apparent from the following description.

According to the present invention, firstly, the above objects and advantages of the present invention are attained by a thermoplastic resin composition comprising:
(A) a thermoplastic polymer having alicyclic groups, and
(B) 0.01 to 5 wt% based on the above thermoplastic polymer of an addition stabilizer which can undergo addition reaction with a radical formed by the cleavage of the above thermoplastic polymer.

According to the present invention, secondly, the above objects and advantages of the present invention are attained by molded products obtained by melt molding the above thermoplastic resin composition of the present invention, for example, molded products for optical use.

According to the present invention, thirdly the above objects and advantages of the present invention are attained by an optical disk substrate on the surface of which only 10 or less crazes are observed per 1 cm² when it is exposed to an atmosphere of 80° C and 85 RH% for 500 hours and which is essentially made from a thermoplastic resin having alicyclic groups.

### Brief Description of the Accompanying Drawings

Fig. 1 is an infrared absorption spectrum diagram of samples #1 to #3 obtained by separating a cyclohexane solution of a polymer obtained in Reference Example 1;
Fig. 2 is a microscopic Raman spectrum diagram of crazes observed through a microscope after a disk substrate obtained in Comparative Example 1 is exposed to an atmosphere of 80° C and 85 RH% for 500 hours to promote its deterioration;
Fig. 3 is a Raman spectrum diagram used for comparison in Reference Example 2; and
Fig. 4 is an UV-VIS spectrum diagram of sample X and sample Y obtained in Reference Example 3.

### Detailed Description of the Preferred Embodiments

### (A) thermoplastic polymer having alicyclic groups

Thermoplastic polymers having alicyclic groups used in the present invention are roughly divided into thermoplastic polymers having alicyclic groups in the main chain and thermoplastic polymers having alicyclic groups in the side chain. The thermoplastic polymers of the former type are further divided into addition polymers and hydrogenated ring-open polymers.

The addition polymers are obtained by copolymerizing a cyclic olefin with ethylene and/or an α-olefin. They are disclosed by JP-A 60-168708, JP-A 61-115916, JP-A 61-221206 and JP-A 61-292601. Examples of the cyclic olefin include preferably cyclic monoolelfins such as norbornene, 5-phenylnorbornene, tetracyclo[4.4.0.1^{2,5}.1^{7,10}]-3-dodecene and 8-phenyl tetracyclo[4.4.0.1^{2,5}.1^{7,10}]-3-dodecene; and cyclic dienes such as cyclopentadiene, dicyclopentadiene, norbornadiene, 5-ethylidenenorbornene, norbornadiene, 8-ethylidene tetracyclo[4.4.0.1^{2,5}.1^{7,10}]-3-dodecene and 8-isopropylidene tetracyclo[4.4.0.1^{2,5}.1^{7,10}]-3-dodecene. Out of these, norbornene, tetracyclo[4.4.0.1^{2,5}.1^{7,10}]-3-dodecene and dicyclopentadiene are particularly preferred from viewpoints of easy acquisition of a raw material and heat resistance of a polymer. Since the formed polymer contains a C=C double bond when a cyclic diene is used out of these, the addition polymer is preferably further hydrogenated in the same manner as the process for producing a hydrogenated ring-open polymer to be described hereinafter.

Examples of the α-olefin include α-olefins having 3 to 20 carbon atoms such as propylene, 1-butene, 1-pentene, 1-hexene, 4-methyl-1-pentene, 1-heptene and 1-dodecene. As the monomer to be copolymerized with a cyclic olefin or cyclic diolefin, the most preferred is ethylene from the viewpoint of reactivity. Propylene is also advantageously used. They maybe used alone or in combination of two or more. The addition polymer can be generally synthesized in the presence of a Ziegler catalyst or metallocene catalyst with ease.

The hydrogenated ring-open polymers are obtained by hydrogenating a ring-open polymer which is a precursor. The ring-open polymer is obtained by polymerizing a cyclic olefin or cyclic diolefin in the presence of a metathesis catalyst. They are disclosed in JP-A 60-26024, JP-A 63-218726, JP-A 2-133413 and JP-A 3-109418. The monomer used is preferably a cyclic monoolefin such as norbornene, 5-phenylnorbornene, tetracyclo[4.4.0.1^{2,5}.1^{7,10}]-3-dodecene or 8-phenyltetracyclo[4.4.0.1^{2,5}.1^{7,10}]-3-dodecene; or cyclic diene such as dicyclopentadiene, norbornadiene, 5-ethylidene norbornene, 5-isopropylidene norbornene, 8-ethylidene tetracyclo[4.4.0.1^{2,5}.1^{7,10}]-3-dodecene or 8-isopropylidene tetracyclo[4.4.0.1^{2,5}.1^{7,10}]-3-dodecene. Unlike the addition polymers, the ring-open polymers inevitably contain a C=C double bond. The polymers containing a C=C double bond lacks thermochemical stability and cannot stand practical use. Therefore, they are further hydrogenated in the presence of a hydrogenation catalyst. Examples of the hydrogenation catalyst include solid catalysts having a precious metal such as nickel, palladium, platinum, cobalt, ruthenium or rhodium, or a compound such as an oxide, salt or complex thereof carried on a porous carrier such as carbon, alumina, silica, silica·alumina or diatomaceous earth, and homogeneous catalysts such as halides, acetylacetonate complexes, carboxylate complexes, naphthate complexes, trifluoroacetate complexes and stearate complexes of a transition metal such as vanadium, chromium, manganese, iron, ruthenium, cobalt, rhodium, nickel or palladium,.

As the other type of thermoplastic polymers having alicyclic groups used in the present invention are hydrogenated styrene polymers having cyclohexyl groups in the side chains.

The hydrogenated styrene polymers used in the present invention are obtained by hydrogenating the aromatic group of a corresponding styrene polymer and a C=C double bond contained in a comonomer component in addition to that of a styrene component in the case of a copolymer. Polyvinyl cyclohexane disclosed in JP-B 7-114030 (the term "JP-B" as used herein means an "examined Japanese patent publication") is such an example. Examples of the styrene monomer unit constituting this styrene polymer include styrene, α-methylstyrene, p-methylstyrene and vinylnaphthalene. Out of these, styrene is the most preferably used from the viewpoints of easy acquisition and the physical properties of the obtained polymer. These monomers may be used alone or in combination of two or more.

Other monomer copolymerizable with the styrene monomer is preferably used to improve physical properties. As this monomer is generally used a conjugated diene. Examples of the conjugated diene include 1,3-butadiene, isoprene, 2,3-dimethyl-1,3-butadiene, 1,3-pentadiene and 1,3-hexadiene. Out of these, 1,3-butadiene and isoprene are preferred from the viewpoints of polymerizability and economy. They may be used alone or in combination of two or more. By introducing the comonomer component, the mechanical properties of the hydrogenated styrene polymer of interest can be greatly improved without impairing transparency. The introduction rate (copolymerization rate) of the comonomer component is preferably 1 to 30 wt%, particularly preferably 3 to 20 wt% based on the total of all the monomers. Although the amount of the conjugated diene component is preferably as large as possible to improve mechanical properties such as impact resistance, if it is too large, transparency and heat resistance (glass transition temperature, thermal deformation temperature) will lower disadvantageously. When the amount of the conjugated diene component is smaller than 1 wt%, its effect of improving toughness becomes small disadvantageously.

The method of producing these styrene polymers is not particularly limited and general radical polymerization, anion polymerization and cation polymerization may be employed. According to the bonding manner of a styrene copolymer obtained by introducing a comonomer component into a styrene polymer, there are a random copolymer, block copolymer and radial block copolymer. Out of these, a block copolymer is particularly preferred when importance is attached to heat resistance and a radial block copolymer (star-like block copolymer, that is, polymer having three or more block chains extending from the core) is particularly preferred when importance is attached to flowability at the time of molding. A suitable copolymer may be selected from among them according to application purpose.

The method of hydrogenating a styrene polymer is not particularly limited in the present invention and any method is acceptable if the aromatic group and the C=C double bond of the polymer can be hydrogenated efficiently. A hydrogenation reaction is generally carried out at a high temperature in an inert solvent under hydrogen pressure in the presence of a hydrogenation catalyst. As the hydrogenation catalyst is used a solid catalyst having a precious metal such as nickel, palladium, platinum, cobalt, ruthenium or rhodium, or a compound such as an oxide, salt or complex thereof carried on a porous carrier such as carbon, alumina, silica, silica·alumina or diatomaceous earth. Out of these, a solid catalyst having nickel, palladium, rhodium or platinum carried on alumina, silica, silica.alumina or diatomaceous earth is preferred because it has high activity. The amount of the hydrogenation catalyst which depends on its catalytic activity is preferably in the range of 0.5 to 40 wt% based on the styrene polymer.

The inert solvent used in the hydrogenation reaction is preferably a hydrocarbon-based solvent. Illustrative examples of the hydrocarbon-based solvent include aliphatic hydrocarbons such as pentane, hexane, heptane, octane and decane; alicyclic hydrocarbons such as cyclopentane, cyclohexane, methylcyclohexane, cyclooctane and decalin; and aromatic hydrocarbons such as benzene, toluene, xylene and tetralin. Out of the above hydrocarbon-based solvents, cyclohexane and methylcyclohexane are preferred because they are inactive with a hydrogenation catalyst and advantageous in terms of solubility, reactivity and economy.

In addition to the above hydrocarbon-based solvent, a polar solvent may be used to control the hydrogenation reaction and suppress decomposition during the hydrogenation reaction. Preferred examples of the polar solvent include chain, branched and cyclic ethers such as tetrahydrofuran, dioxane, diethylene glycol, dimethyl ether, diethyl ether and methyl-tert-butyl ether. These hydrocarbon-based solvents and polar solvents may be used alone or as a mixture of two or more.

The concentration of a reaction solution containing the above styrene-based polymer is preferably 5 to 40 wt%, more preferably 10 to 30 wt%. When the concentration is lower than 5 wt%, productivity drops and when the concentration is higher than 40 wt%, it is difficult to control the hydrogenation reaction.

As for the hydrogenation reaction conditions, in general, the hydrogen pressure is 30 to 250 kgf/cm² (about 2.9 to 24.5 Pa) and the reaction temperature is 70 to 250°C. When the reaction temperature is too low, the reaction hardly proceeds and when the reaction temperature is too high, the molecular weight is readily reduced by the breakage of the molecular chain. To prevent a reduction in molecular weight caused by the breakage of the molecular chain and promote the reaction smoothly, the hydrogenation reaction is preferably carried out at a suitable temperature and hydrogen pressure which are determined by the type and concentration of the catalyst used, the concentration of the solution of the styrene polymer and the molecular weight of the styrene polymer.

The method of purifying the thus obtained hydrogenated styrene polymer is not particularly limited and a general method can be employed. The hydrogenated styrene polymer can be generally obtained by removing the catalyst from the solution of the hydrogenated styrene polymer obtained in the step of the hydrogenation reaction by centrifugation or filtration and distilling off the solvent from the obtained filtrate. In the composition of the present invention which is suitably used as an optical material (molding material), the contents of the residual metal components in the composition must be reduced as much as possible and the amount of the residual catalytic metal is preferably 10 ppm or less, more preferably 5 ppm or less, much more preferably 2 ppm or less.

As for the molecular weight of the hydrogenated styrene polymer used in the present invention, the weight average molecular weight (Mw) in terms of polystyrene measured by GPC (Gel Permeation Chromatography) of the polymer is 30,000 to 1,000,000, more preferably 50,000 to 500,000, much more preferably 80,000 to 400,000 when the mechanical strength of a molded product and moldability are taken into consideration. When the weight average molecular weight (Mw) is lower than 30,000, mechanical strength becomes insufficient and when the weight average molecular weight is higher than 1,000,000, melt viscosity becomes too high and flowability becomes insufficient, thereby making molding difficult. When this is expressed by reduced viscosity which is one of molecular weight indices, the reduced viscosity (ηsp/C) measured in a toluene solution having a concentration of 0.5 g/dL at 30°C is 0.1 to 10 dL/g, preferably 0.2 to 3 dL/g, more preferably 0.3 to 1.0 dL/g.

### (B) addition stabilizer

The addition stabilizer used in the present invention can addition react with a radical formed by the cleavage of the thermoplastic polymer (A).

It is generally said that the thermal decomposition of the thermoplastic polymer (A) is started by the formation of a free radical or radical in the main chain caused by the uniform cleavage of a weak C-H bond in the molecular chain. The molecular chain is broken by the further cleavage of a C-C bond containing the free radical or radical. The resulting cleaved chain (fragment) contains a free carbon atom (terminal C-radical) at a terminal. This terminal C-radical is very active and takes a hydrogen atom as a free hydrogen atom (H-radical) from a weak C-H bond nearby to be stabilized. However, a new free radical is formed in the main chain at the same time, thereby causing C-C cleavage. By repetitions of the above series of elementary reactions, chain cleavage proceeds.

It is said that a hindered phenol used as a typical stabilizer releases a hydrogen radical to stabilize a C-radical formed in the main chain or at a terminal of a cleaved chain, thereby making it possible to suppress thermal decomposition by adding a hindered phenol-based stabilizer and a further reduction in molecular weight. However, a decomposed product of the hindered phenol which has contributed to stabilization grows in quantity, agglomerates and separates out in a molded product as fine particles. Therefore, the amount of the fine particles is much larger than when the hindered phenol is not added. As generally said, the hindered phenol-based stabilizer releases the hydrogen atom of a phenolic hydroxyl group into a free radical in a polymer formed by thermal decomposition as a free radical to be reacted with the free radical in the polymer for stabilization in order to suppress the subsequent chain decomposition reaction. However, the other free radical of the hindered phenol remains in the molded product as a decomposed product of the hindered phenol at the same time.

It has been found that when a molded product of the thermoplastic polymer (A) containing the hindered phenol is exposed to an atmosphere of high temperature and high humidity for a long time, crazes are generated from nuclei which are formed by a decomposed product derived from an unhydrogenated product and a decomposed product of the hindered phenol contained in the polymer in trace amounts. Since the crazes are much larger than the nuclei, if there is a trace amount of fine particles, they are expanded.

Also, a reduction in molecular weight caused by chain cleavage cannot be avoided by the addition of the hindered phenol as described above.

It has been made clear that the addition stabilizer (B) used in the present invention does not generate a decomposed product of the hindered phenol unlike the hindered phenol and reacts with a plurality of cleaved terminals of the polymer to suppress even a reduction in molecular weight caused by chain cleavage when it is polyfunctional.

The addition stabilizer (B) used in the present invention is preferably a phenolic compound having a (meth)acrylate group.

Examples of the addition stabilizer (B) include a compound obtained by converting 35 to 65 mol% of the hydroxyl groups of a chain phenol-aldehyde condensate represented by the following formula (1) into (meth)acryloyloxy groups: 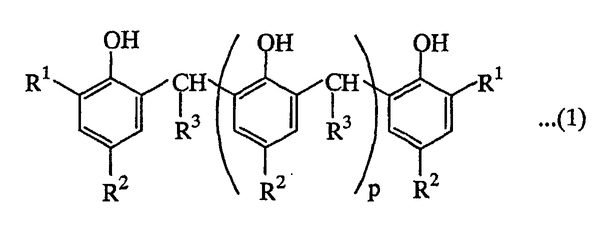 wherein R¹, R² and R³ are each independently a hydrogen atom or alkyl group having 1 to 10 carbon atoms and p is an integer of 0 to 13, with the proviso that R¹'s, R²'s and R³'s may be the same or different,
a compound obtained by converting 35 to 65 mol% of the hydroxyl groups of a cyclic phenol-aldehyde condensate represented by the following formula (2) into (meth)acryloyloxy groups: 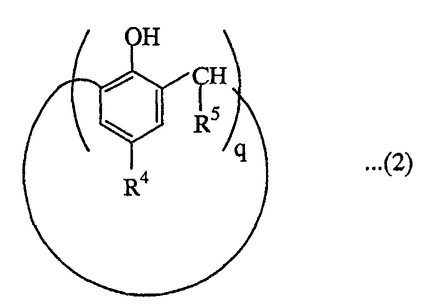 wherein R⁴ and R⁵ are the same or different and each a hydrogen atom or alkyl group having 1 to 10 carbon atoms and q is an integer of 4 to 15,
and a compound obtained by converting 35 to 65 mol% of the hydroxyl groups of a cyclic phenol-aldehyde condensate represented by the following formula (3) into (meth)acryloyloxy groups: 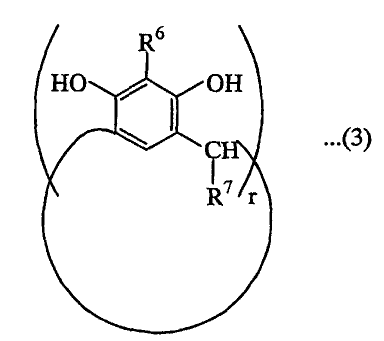 wherein R⁶ and R⁷ are the same or different and each a hydrogen atom or alkyl group having 1 to 10 carbon atoms and r is an integer of 4 to 15.

In the present invention, the above compounds may be used alone or in combination of two or more as the addition stabilizer.

The phenol-aldehyde condensates represented by the above formulas (1), (2) and (3) are condensates in which a phenol and an aldehyde are condensed at an ortho-position with respect to the hydroxyl group of the phenol and the average number of the residual phenol groups in one molecule is 2 to 15 (p = 0 to 13). The phenol-aldehyde condensates are roughly divided into chain phenol-aldehyde condensates of the formula (1) and cyclic phenol-aldehyde condensates of the formulas (2) and (3).

As the phenol is used a phenol whose two ortho positions (called "o-position" and "o'-position") with respect to the hydroxyl group are not substituted excluding a phenol for the terminal residual phenol group of the phenol-aldehyde condensate. One of the reasons for this is that the o-position, o'-position and para-position (p-position) have high activity in a reaction for substituting a parent electron for a phenol and another reason is that the residual phenol group adjacent to the (meth)acryl group of the (meth)acrylated phenol in the present invention is situated at a spatially advantageous position through a bridging group derived from an aldehyde (to be simply referred to as "bridging group" hereinafter) formed by a condensation reaction between it and an aldehyde. This structure exhibits a great radical stabilization effect.

As the phenol is preferably used a non-substituted or p-position substituted phenol. Examples of the phenol include non-substituted phenol and p-substituted phenols having an alkyl group having 1 to 10 carbon atoms such as p-methylphenol, p-ethylphenol, p-isopropylphenol, p-n-butylphenol, p-sec-butylphenol, p-tert-butylphenol, p-1,1-dimethylpropylphenol, p-octylphenol and p-decylphenol. Out of these, p-cresol(p-methylphenol), p-tert-butylphenol and p-1,1-dimethylpropylphenol are particularly preferred from the viewpoint of the easy acquisition of raw materials.

Meanwhile, the phenol located at both terminals of the chain phenol-aldehyde condensate may be a phenol one of the ortho-positions of which is not substituted. From the viewpoint of radical stabilization mechanism, one of the ortho-positions is preferably substituted by a steric-hindrance substituent. When this is taken into account, besides the above phenols, preferred phenols are o-position substituted phenols such as 2-methylphenol (o-cresol), 2,4-dimethylphenol, 2-tert-butyl-4-methylphenol, 2,4-di-tert-butylphenol, 2-(1,1-dimethylpropyl)-4-methylphenol and 2,4-di(1,1-dimethylpropyl)phenol. Out of these, 2-tert-butyl-4-methylphenol, 2,4-di-tert-butylphenol, 2-(1,1-dimethylpropyl)-4-methylphenol and 2,4-(1,1-dimethylpropyl)phenol are particularly preferred.

As another type of phenols used in the present invention are resorcin (resorcinol) type bifunctional phenols. They are preferred because two different ortho-positions with respect to the two hydroxyl groups of resorcin are active in a parent electron substitution reaction and the (meth)acryl group and the hydroxyl group are situated at spatially advantageous positions through a bridging group in the (meth)acrylated phenol-aldehyde condensate after (meth)acrylation. The resorcin type bifunctional phenols include resorcin and 5-methylresorcin. In this case, what has a high steric hindrance group at the 5-position is not preferred because it prevents a condensation reaction.

The aldehyde used in the present invention is an aliphatic aldehyde having 1 to 11 carbon atoms. The aldehyde is selected in consideration of reactivity, easy acquisition and the spatial location of the hydroxyl group spaced apart from the (meth)acryl group by a bridging group. Specific examples of the aldehyde include formaldehyde, paraformaldehyde, acetaldehyde, propionaldehyde, butanal, isobutanal, pivaloaldehyde and octanal. Out of these, formaldehyde, paraformaldehyde, acetaldehyde, butanal and pivaloaldehyde are particularly preferred.

The average number of the residual phenol groups in one molecule of the phenol-aldehyde condensate in the present invention is 2 to 15, preferably 2 to 12, more preferably 2 to 10. The (meth)acrylated phenol-aldehyde condensate obtained by (meth)acrylating this phenol-aldehyde condensate exhibits its function the most effectively as a polyfunctional addition stabilizer when the residual phenol groups and the residual (meth) acrylated phenol groups adjacent thereto exist in pairs. As the polyfunctional addition stabilizer, the average number of the residual phenol groups in one molecule of the phenol-aldehyde condensate is preferably 4 to 15, more preferably 4 to 12, particularly preferably 4 to 10. When the average number of the residual phenol groups is smaller than 4, the average number of pairs is smaller than 2, the function of the polyfunctional addition stabilizer lowers disadvantageously. In other words, chain extendability lowers disadvantageously. When the average number of the residual phenol groups is larger than 15, the probability of forming the above pairs in one molecule does not increase and the molecular weight rises, whereby the amount of the addition stabilizer for exhibiting its function the most effectively for the resin increases disadvantageously.

In the above formula (1), R¹ is particularly preferably a tert-butyl group or 1,1-dimethylpropyl group. R² is particularly preferably a methyl group, tert-butyl group or 1,1-dimethylpropyl group. R³ is particularly preferably a hydrogen atom, methyl group, propyl group or tert-butyl group. The average value of p is 0 to 13, preferably 0 to 10, more preferably 0 to 8. In the case of a polyfunctional addition stabilizer, the average value of p is preferably 2 to 13, more preferably 2 to 10, particularly preferably 2 to 8.

In the above formula (2), R⁴ and R⁵ are particularly preferably the same as R² and R³ in the above formula (1), respectively. q is 4 to 15, preferably 4 to 12, much more preferably 4 to 10.

In the above formula (3), R⁷ is particularly preferably the same as R³ in the above formula (1). R⁶ is a hydrogen atom or alkyl group having 1 to 10 carbon atoms, particularly preferably a hydrogen atom or methyl group. r is 4 to 15, preferably 4 to 12, more preferably 4 to 10.

In the present invention, 35 to 65 mol%, preferably 40 to 60 mol%, more preferably 45 to 55 mol% of the hydroxyl groups of the phenol-aldehyde condensate are (meth)acrylated. As described above, by (meth)acrylation, the phenol-aldehyde condensate fulfills its function as an addition stabilizer. The reason that some phenolic hydroxyl groups are kept is that when a free radical reacts with a (meth)acryl group, a new free radical is formed at the α-position. This radical has high activity and is able to take a hydrogen radical from a saturated cyclic compound. However, when a phenolic hydroxyl group is existent near the radical, the radical takes a hydrogen atom from the hydroxyl group as a free radical to be stabilized. Since the formed O-radical has low activity, it has no ability to induce a further chain reaction. Therefore, it is preferred that the (meth)acryl groups and the hydroxyl groups should be balanced. From this point of view, when the (meth)acrylation rate is lower than 35 %, the (meth)acryl groups are not enough for the hydroxyl groups and when the (meth)acrylation rate is higher than 65 %, the (meth)acryl groups are more than enough for the hydroxyl groups . In both cases, the effect lowers.

The addition stabilizer (B) based on the chain phenol-aldehyde condensate of 'the formula (1) in which p = 0 is preferably represented by the following formula (1'): 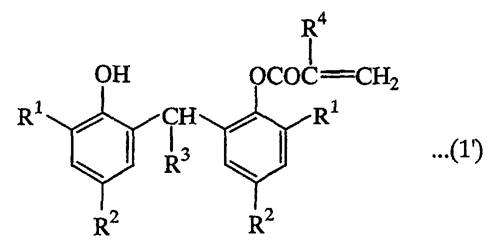 wherein R¹, R² and R³ are as defined in the above formula (1) and R⁴ is a hydrogen atom or methyl group.

The addition stabilizer represented by the above formula (1') stabilizes a C-radical formed at a terminal of the cleaved chain of the polymer without forming a decomposed product.

Illustrative examples of the alkyl group represented by R¹ to R³ in the above formula (1') include methyl, ethyl, n-propyl, isopropyl, n-butyl, sec-butyl, isobutyl, tert-butyl and 1,1-dimethylpropyl. R¹ is preferably a bulky alkyl group which becomes steric hindrance, such as isopropyl, sec-butyl, tert-butyl or 1,1-dimethylpropyl from the viewpoints of effect and production ease. Out of these, tert-butyl and 1,1-dimethylpropyl are particularly preferred. R² is preferably methyl, tert-butyl or 1,1-dimethylpropyl from the viewpoint of ease for production. However, since methyl readily causes a side reaction accompanied by the extraction of a hydrogen atom, R² is more preferably tert-butyl or 1,1-dimethylpropyl. R³ is preferably an alkyl group which hardly becomes steric hindrance, such as methyl, ethyl, propyl or n-butyl from the viewpoint of production. R⁴ is a hydrogen atom or methyl group.

The compound represented by the above formula (1') is commercially available, for example, "the Sumilizer GM" (trade name) and "Sumilizer GS" (trade name) of Sumitomo Chemical Co., Ltd.

These addition stabilizers may be used alone or in combination of two or more. A hindered phenol-based stabilizer such as "Irganox 1010" (trade name) and "Irganox 1076" (trade name) (of Ciba Specialty Chemicals Co., Ltd.) or an antioxidant such as "Irgafos 168" (trade name) (of Ciba Specialty Chemicals Co., Ltd.) may be used as required.

The phenol-aldehyde condensate for the addition stabilizer in the present invention may be synthesized by a known method per se.

The chain phenol-aldehyde condensates are generally obtained by reacting a phenol with an aldehyde preferably in a solvent under an acidic or basic condition by heating. Preferred examples of the solvent, which depends on the types of the phenol and the aldehyde, include water, chain and cyclic saturated hydrocarbons such as hexane, heptane, cyclopentane, cyclohexane and methylcyclohexane, aromatic hydrocarbons such as benzene, toluene and xylene, alcohols such as methanol, ethanol and isopropanol, chain and cyclic ethers such as ethyl ether, methyl-tert-butyl ether, ethylene glycol dimethyl ether, diethylene glycol dimethyl ether, tetrahydrofuran, 1,3-dioxolan and dioxane, esters such as ethyl acetate, butyl acetate and isobutyl acetate, hydrocarbon halides such as methylene chloride and chloroform, and nitrogen-containing solvents such as acetonitrile and nitromethane. To promote the reaction, an inorganic strong acid such as hydrogen chloride, hydrochloric acid, sulfuric acid, nitric acid or phosphoric acid, or an organic acid such as acetic acid, dichloroacetic acid, methanesulfonic acid or p-toluenesulfonic acid is preferably used. A basic compound such as sodium hydroxide, potassium hydroxide, ammonia, calcium hydroxide or barium hydroxide is also preferably used. The reaction temperature is generally 30 to 200° C, preferably 50 to 150° C. The reaction time is 1 to 50 hours, preferably 2 to 25 hours.

The cyclic phenol-aldehyde condensates may be easily synthesized by methods disclosed in Journal of Organic Chemistry, vol. 54, p.1305 (1989), Organic Synthesis, vol. 68, p.234 (1999) and the like. The phenol-aldehyde condensate obtained in the present invention may be directly purified before use or may be fractionated as required before use. When a single compound is necessary, a fractionating technology based on liquid chromatography is preferably used.

In order to obtain a (meth)acrylated phenol-aldehyde condensate by (meth)acrylating the obtained phenol-aldehyde condensate in the present invention, a condensation reaction between the phenol-aldehyde condensate and (meth) acrylic acid or an active derivative thereof is used. An active derivative such as a (meth)acrylic acid ester, (meth)acrylic acid chloride or (meth)acrylic acid anhydride is generally used and (meth)acrylic acid chloride is particularly preferably used. The reaction is carried out in an inert solvent under cooling, normal temperature or heating. Examples of the inert solvent include chain and cyclic saturated hydrocarbons such as hexane, heptane, cyclopentane, cyclohexane and methylcyclohexane; aromatic hydrocarbons such as benzene, toluene and xylene; chain, cyclic and aromatic ethers such as ethyl ether, methyl-tert-butyl ether, ethylene glycol diethyl ether, diethylene glycol diethyl ether, tetrahydrofuran, dioxane, 1,3-dioxolan and anisole; esters such as methyl acetate, ethyl acetate and isobutyl acetate; chain and cyclic ketones such as acetone, methyl ethyl ketone, methyl isobutyl ketone and cyclohexanone; high-boiling polar solvents such as dimethylformamide, dimethylacetamide, N-methylpyrrolidone and dimethyl sulfoxide; pyridines such as pyridine and quinoline; hydrocarbon halides such as methylene chloride and chloroform; and nitrogen-containing solvents such as acetonitrile and nitromethane. When a (meth)acrylic acid chloride or (meth)acrylic acid anhydride is used, an acid receptor such as a tertiary amine exemplified by trimethylamine, triethylamine, tri-n-butylamine and pyridine is preferably used in combination. The reaction which greatly depends on the type of the active derivative used and the solvent is generally carried out at 0 to 150° C, preferably 10 to 100° C. The reaction time is 1 to 50 hours, preferably 2 to 25 hours.

In the present invention, the (meth)acrylated phenol-aldehyde condensate may also be obtained by reacting a phenol and (meth) acrylated phenol with an aldehyde by heating. The (meth)acrylated phenol can be synthesized by (meth)acrylating a phenol as described above and the (meth)acrylated phenol-aldehyde condensate can be synthesized by the same method as the synthesis of the phenol-aldehyde condensate.

As obvious from above, when the addition stabilizer used in the present invention is polyfunctional, that is, the addition stabilizer has at least two (meth)acryloyl groups in the molecule, it has the function of stabilizing the cleaved terminals of the polymer and also the function of a chain extender for connecting a plurality of cleaved terminals of the polymer for stabilization. Therefore, the addition stabilizer used in the present invention comprises a (meth)acrylated compound of the above formula (1) in which p = 0, having the function of stabilizing the cleaved terminals of the polymer, and a (meth)acrylated compound of the above formula (1) in which p is an integer of 1 to 15, having the function of a chain extender, so as to exhibit these functions effectively.

That is, a combination of the compound of the above formula (1) in which p is 0 and the compound of the above formula (1) in which p is an integer of 1 to 13 is advantageously used as the addition stabilizer (B) in the present invention.

In the present invention, the addition stabilizer (B) is used in an amount of 0.01 to 5 wt%, preferably 0.05 to 3 wt%, more preferably 0.1 to 2 wt% based on the thermoplastic polymer (A). Below the above range, its effect becomes unsatisfactory and above the range, its effect reaches saturation and the agglomeration of a thermally decomposed product of the addition stabilizer which did not contribute to the stabilization of thermal decomposition occurs disadvantageously.

In the present invention, a polyfunctional (meth)acrylate compound and polyfunctional (meth)allyl compound of an aliphatic or alicyclic polyol may be further used as a chain extender. These chain extenders may be used alone or in combination of two or more.

Preferably, the polyfunctional (meth) acrylate compound has 2 to 10 (meth) acryloyloxy groups in one molecule. Specific examples of the polyfunctional (meth)acrylate compound include aliphatic and alicyclic bifunctional (meth)acrylate compounds such as 1,2-ethylene glycol di(meth)acrylate, 1,6-hexanediol di(meth)acrylate, neopentylene glycol di(meth)acrylate, diethylene glycol di(meth)acrylate, hydroxypivalic acid neopentyl glycol ester di(meth)acrylate, di(meth)acrylates of a bisphenol-A ethylene oxide adduct and tricyclodecane di(meth)acrylate; and (meth)acrylate compounds having three or more (meth)acryloyloxy groups such as glycerin tri(meth)acrylate, trimethylolpropane tri(meth)acrylate, pentaerythritol tetra(meth)acrylate and dipentaerythritol-penta·hexa(meth)acrylate. Generally speaking, as the number of functional groups increases, the chance of an addition reaction between the (meth)acrylate compound and cleaved chains grows and the molecular weight of an adduct rises. In other words, the effect of suppressing a reduction in molecular weight increases. However, as the molecular weight of the polyfunctional (meth)acrylate compound itself becomes high, unless the amount of the compound is increased, its effect lowers. When the number of carbon atoms of the alcohol component increases, hydrophobic nature becomes strong. In other words, the compatibility of the polyfunctional (meth)acrylate compound with the thermoplastic polymer (A) increases advantageously. A suitable compound may be selected from these polyfunctional (meth)acrylate compounds in consideration of the effect of suppressing a reduction in molecular weight, steam pressure and compatibility with a cyclic polymer. These polyfunctional (meth)acrylate compounds may be used alone or in combination of two or more.

As the polyfunctional (meth)allyl compound may be suitably used a compound having a plurality of allyl ester groups, allyl ether groups or allyl carbonate groups in the molecule. Specific examples of the polyfunctional (meth)allyl compound include diallyl phthalate, diallyl isophthalate, diallyl terephthalate, triallyl trimellitate, tetraallyl pyromellitate, diallyl phthalate-β-polymer, diethylene glycol diallyl carbonate and cyanuric acid triallyl esters.

The above polyfunctional (meth)acrylate compound and/or polyfunctional (meth)allyl compound used as a chain extender in the present invention are/is used in an amount of 0.05 to 5 parts by weight, preferably 0.1 to 3 parts by weight, more preferably 0.2 to 2 parts by weight based on 100 parts by weight of the thermoplastic polymer (A).

The thermoplastic resin composition of the present invention is produced by mixing the thermoplastic polymer (A) having an alicyclic group and the addition stabilizer (B) and optionally the polyfunctional (meth) acrylate compound and/or polyfunctional (meth)allyl compound.

The time of adding the addition stabilizer (B) and the like to the polymer (A) is not particularly limited but it is preferred from the viewpoints of work efficiency and effect that they should be added to a solution of the thermoplastic polymer (B) obtained after the removal of the polymerization catalyst or hydrogenation catalyst. When they are added in this stage, uniform mixing with the polymer is easy and thermal decomposition in the subsequent high-temperature steps such as the solvent removing step (flushing step), pellet formation step and molding step can be suppressed advantageously.

The thermoplastic resin composition of the present invention is characterized in that crazes are rarely generated.

In the present invention, the content of foreign particle in the thermoplastic polymer is preferably low, that is, the number of foreign particles having a particle diameter of 0.5 µm or more is 20,000 or less per g, more preferably 10,000 or less per g, much more preferably 5,000 or less per g. The foreign particles include impurities contained in raw materials, impurities contained in the production step, a gelled product of the polymer, the residual polymerization catalyst and the residual hydrogenation catalyst. When the number of foreign particles having a particle diameter of 0.5 µm or more is larger than 20,000 per g and an optical disk substrate for high-density recording is molded from the polymer, the bit error rate becomes high and the recording and reproduction characteristics of the disk deteriorate.

These foreign particles can be removed by filtration with a filter in each production step and by carrying out the granulating step in a clean room.

When the thermoplastic resin composition of the present invention is injection compression molded into a substrate having a diameter of 12 cm and a thickness of 1.2 mm using a metal mold at a resin temperature of 330° C and a mold temperature of 120°C and this substrate is exposed to an atmosphere of 80° C and 85 RH% for 500 hours, only 10 or less crazes are observed per 1 cm² of the surface of the substrate.

The molding material which is made from the thus obtained thermoplastic resin composition can be formed into various molded products such as a fiber and a plate by known molding methods such as melt molding exemplified by injection molding and extrusion molding, and solution molding exemplified by solution casting and wet film formation. Particularly injection molding can be advantageously used for the production of optical disk substrates. For the molding of optical disk substrates, the resin temperature is 270 to 370° C, preferably 280 to 350° C. When the resin temperature is higher than 370°C, excessive thermal decomposition occurs disadvantageously. When the resin temperature is lower than 270° C, melt flowability lowers disadvantageously. The mold temperature is 60 to 140° C, preferably 70 to 130° C. When the mold temperature is higher than 140°C, the deformation of the molded product occurs disadvantageously. When the mold temperature is lower than 60° C, transferability deteriorates disadvantageously.

According to the present invention, there is provided an optical disk substrate which is mainly made from a thermoplastic resin having an alicyclic group and on the surface of which only 10 or less crazes per 1 cm² are observed when it is exposed to an atmosphere of 80°C and 85 RH% for 500 hours, for example, an optical disk substrate which is made from the thermoplastic resin composition of the present invention.

An optical disk is generally used after a reflection layer and a recording layer are formed on a substrate by sputtering or the like and then a protective layer is formed from an ultraviolet curable resin. Further, an optical disk having a thickness of 0.6 mm used for DVD and the like is produced by joining together two substrates by an ultraviolet curable resin. Although curing is carried out by exposure to ultraviolet radiation, a stabilizer used in the substrate may be colored by exposure to ultraviolet radiation, thereby losing characteristic properties as an optical disk substrate. When information is written or read using a short-wavelength laser beam having a wavelength around 400 nm which has been recently been developed, it is considered that this will cause a serious problem.

Since an optical disk substrate which is made from the resin composition of the present invention is rarely colored by ultraviolet radiation which is applied to cure the ultraviolet curable resin, it is preferably used as an optical disk substrate for use with a laser beam having a short wavelength around 400 nm.

According to the present invention, there is obtained a molding material comprising a polymer having an alicyclic group, which suppresses a reduction in molecular weight at the time of heating such as melt molding as described above. As a result, there is obtained a molded product which is excellent in transparency and heat resistance as well as durability and reliability at a high temperature and high humidity and is rarely colored by exposure to ultraviolet radiation.

Therefore, the molding material of the present invention can be melt molded well at a high temperature and a molded product thereof can be effectively used as a substrate for optical recording media typified by optical disks such as CD, CD-ROM, LD, MO, MD and DVD which must be molded at a high temperature.

### Examples

The following examples and comparative examples are provided for the purpose of further illustrating the present invention but are in no way to be taken as limiting.

Various physical properties in the examples, comparative examples and reference examples were measured in accordance with the following methods.
1) glass transition temperature (Tg): measured at a temperature elevation rate of 20°C/min using the 2920 DSC of TA Instrument Co., Ltd.
2) hydrogenation rate: determined by ¹H-NMR using the JNM-A-400 nuclear magnetic resonance absorption device of JEOL Ltd.
3) molecular weight: The molecular weight in terms of polystyrene was measured by gel permeation chromatography (GPC, Shodex System-11 of Showa Denko K.K.) using THF as a solvent.
4) mass analysis (FD-MASS): using the M-80B mass analyzer of Hitachi, Ltd.
5) reduced viscosity: The reduced viscosity ηsp/c (dL/g) in a toluene solution having a concentration of 0.5 g/dL at 30° C was measured.
6) thermal decomposition GC-MS: The analysis of a thermally decomposed product was carried out by the thermal decomposition GC-MS. Thermal decomposition was carried out in a nitrogen atmosphere at 300°C for 10 minutes using the JHS-100 space gas sampler. Decomposition gas was cooled to -40°C, absorbed by Tenax, desorbed and separated using the Type GCD 18000A gas chromatograph of Yokogawa Electric Corporation. The DB-1701 column was used. The obtained fraction was assigned by the GCD 1800A mass analyzer of Yokogawa Electric Corporation.
7) evaluation of thermal stability of resin: A Koka type flow tester was used. The resin was fed to the nozzle of the Koka type flow tester maintained at 340° C and kept for 5 minutes, and the reduced viscosity ηsp/c of the molten resin obtained by extruding the resin was measured. This was compared with the reduced viscosity of the original resin.
8) evaluation of volatility of stabilizer: A weight reduction curve based on temperature elevation time was obtained using the Rigaku plus TG8120 of Rigaku Denki Co., Ltd. The measurement was carried out at a temperature elevation rate of 80°C/min in a stream of nitrogen. The temperature corresponding to an intersection between a tangent drawn on the curve before the occurrence of a sudden weight reduction and a tangent drawn on the curve of the maximum gradient area is defined as evaporation start temperature.
9) contents of residual metals in polymer: determined by ICP emission spectral analysis.
10) total light transmission: using the ultraviolet-visible spectrograph (UV-240) of Shimadzu Corporation.
11) haze value: using the UDH-20D automatic digital haze meter of Nippon Denshoko Kogyo Co., Ltd.
12) water absorption coefficient: measured in accordance with ASTM D-570.
13) birefringence index: Retardation was measured using the ADR200B of Oak Seisakusho Co., Ltd.
14) infrared absorption spectrum: using the FTS-65A FT-IR of BIO-RAD Co., Ltd. and the IR-PLAN microscope of SPECTRA-TECH Co., Ltd.
15) microscopic Raman spectrum: using the T-64000 microscopic RAMAN device of JOBIN-YVON Co., Ltd. and an argon ion laser (514.5 nm) as a light source.
16) UV exposure: exposed to 60 mW/cm² of UV for 5 seconds using the P150 ultraviolet curing coater of FUSION SYSTEMS Co., Ltd. Transmission at 400 nm was measured with the U-3200 spectrophotometer of Hitachi, Ltd.

### Example 1

After the inside of a 10-liter stainless steel autoclave equipped with agitating elements was fully dried and substituted with nitrogen, 750 g of polystyrene (Mw = 2.8 x 10⁵), 118 g of Ni/silica·alumina catalyst, 2,200 g of cyclohexane and 1,500 g of methyl-tert-butyl ether were fed to the autoclave. After the inside of the reactor was fully substituted with hydrogen, a hydrogen pressure of 100 kgf/cm² (about 9.8 MPa) was applied to carry out a hydrogenation reaction at 180° C for 6 hours under agitation. After the end of the reaction, when the obtained suspension (slurry) was filtered with a membrane filter having an opening diameter of 0.1 µm (Fluoropore of Sumitomo Electric Industries, Ltd.) under pressure, an achromatic transparent solution was obtained. To this filtrate was added 3.1 g (0.4 wt% based on the hydrogenated styrene polymer) of the Sumilizer GS (of Sumitomo Chemical Co., Ltd.) (in the formula (1'), R¹ = R² = 1,1-dimethylpropyl group, R³ = methyl group, R⁴ = hydrogen atom) and then the solvent was distilled off to obtain a hydrogenated styrene polymer.

The hydrogenation rate determined by ¹H-NMR of the polymer was 99.3 %. The reduced viscosity (ηsp/c) measured in a toluene solution having a concentration of 0.5 g/dL at 30° C of the polymer was 0.47 dL/g. Further, it was found by ICP emission spectral analysis that the residual metals were contained in the resin in an amount of 1 ppm or less each, that is, the content of Ni was 0.18 ppm, the content of Al was 0.28 ppm, and the content of Si was 0.23 ppm. The glass transition temperature (Tg) measured by DSC of the polymer was 149° C.

Thereafter, the hydrogenated styrene polymer was ground to obtain granules for molding. 5 batches of the obtained granules were collected to injection mold an achromatic transparent disk substrate (a diameter of 12 cm and a thickness of 0.6 mm) at a resin temperature of 330° C and a mold temperature of 120° C using a DVD stamper. The disk substrate had an extremely low water absorption coefficient of 0.01 wt% or less and extremely high transparency with a total transmission of 91 % and a haze value of 1.2 %. The birefringence index of the peripheral portion of the obtained disk substrate was very low at 5 nm. In other words, the disk substrate showed high optical isotropy.

The disk substrate had a transmission at 400 nm of 90 %. When the disk substrate was exposed to 60 mW/cm² of ultraviolet radiation for 2 seconds and the transmission at 400 nm of the disk substrate was measured again, it was 85 %.

After the disk substrate was exposed to an atmosphere of 80° C and 85 RH% for 500 hours before exposure to ultraviolet radiation to promote its deterioration, the number of crazes was counted by observing through a microscope. As a result, the number of crazes on the surface of the substrate was 1 per 1 cm².

### Comparative Example 1

3.1 g (0.4 wt% based on the hydrogenated styrene polymer) of the Irganox 1010 (of Ciba Specialty Chemicals Co., Ltd.) which is a typical hindered phenol and 3.1 g (0.4 wt% based on the hydrogenated styrene polymer) of the Irgafos 168 (of Ciba Specialty Chemicals Co., Ltd.) which is a typical antioxidant were added to the achromatic transparent solution after filtration obtained in Example 1 and then the solvent was distilled off to obtain a hydrogenated styrene polymer. The hydrogenation rate determined by ¹H-NMR of the polymer was 99.3 %. The reduced viscosity (ηsp/c) measured in a toluene solution having a concentration of 0.5 g/dL at 30° C of the polymer was 0.47 dL/g.

The above hydrogenated styrene polymer was ground to obtain a granules for molding. 3 batches of the obtained granules were collected to injection mold an achromatic transparent disk substrate (a diameter of 12 cm and a thickness of 0.6 mm) at a resin temperature of 330° C and a mold temperature of 120° C using a DVD stamper.

After the disk substrate was exposed to an atmosphere of 80° C and 85 RH% for 500 hours to promote its deterioration, the number of crazes was counted by observing through a microscope. As a result, the number of crazes was 100 or more per 1 cm².

### Reference Example 1

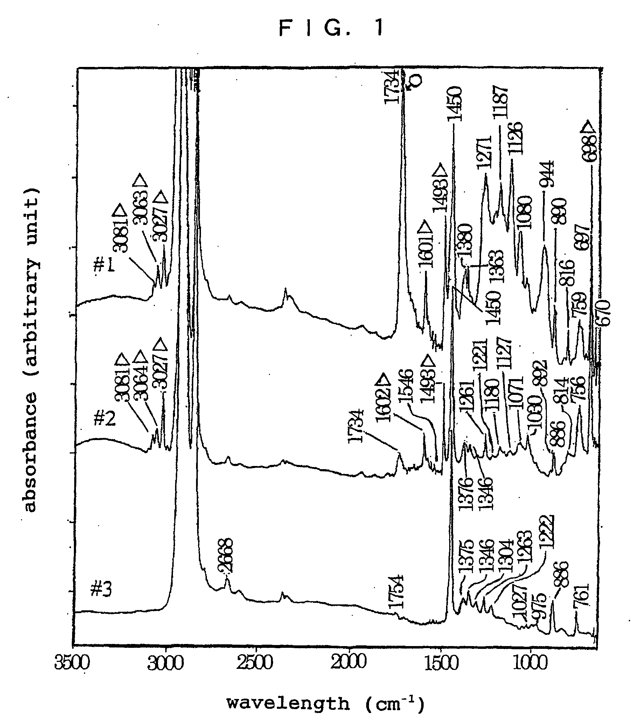

A 20 % solution was prepared by dissolving the hydrogenated styrene resin obtained in Comparative Example 1 in cyclohexane. This solution was slightly cloudy. This solution was filtered with a membrane filter having an opening diameter of 0.45 µm to separate a precipitate (P1) and a filtrate (F1). The precipitate (P1) was extracted and cleaned with chloroform on the filter to separate a filtrate (F2) and an undissolved product (P2). The filtrate (F2) was dropped on a preparation to evaporate, dry up and distill off the solvent, and a trace amount of the obtained solid was designated as sample #1. A trace amount of a solid obtained by cleaning the undissolved product (P2) with methanol was designated as sample #2. Meanwhile, the solvent was distilled off from the filtrate (F1) to obtain a large amount of sample #3. The infrared absorption spectra of the obtained samples #1, #2 and #3 were measured. The results are shown in Fig. 1. Δ in Fig. 1 shows a peak derived from polystyrene and ○ shows a peak based on a carbonyl group.

As obvious from Fig. 1, the sample #3 gave the typical spectrum of polystyrene hydride. In contrast to this, a peak derived from polystyrene was observed in addition to a peak derived from polystyrene hydride in the sample #2. This indicates that the sample #2 comprised an unhydrogenated styrene component. Meanwhile, a strong absorption based on a carbonyl group was observed at 1,734 cm⁻¹ in addition to peaks based on polystyrene hydride and an unhydrogenated styrene component in the sample #1. It is considered that this is derived from a decomposed product of the Irganox 1010. It is obvious from the fact that the Irganox 1010 dissolves in cyclohexane that this is derived not from the Irganox 1010 itself but from its decomposed product.

It can be concluded from the above results that the above cloudy substance comprised a decomposed product of the polymer containing an unhydrogenated styrene component and a decomposed product of the Irganox 1010.

### Reference Example 2

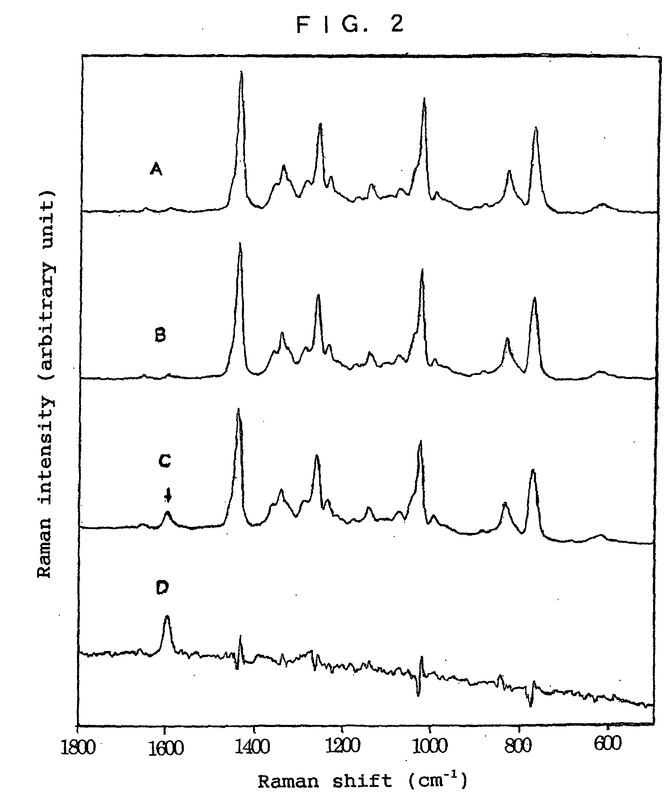

After the optical disk substrate obtained in Comparative Example 1 was exposed to an atmosphere of 80° C and 85 RH% for 500 hours to promote its deterioration, it was observed through a microscope. As a result, saber-like crazes extending from nuclei as large as 1 µm were seen. The crazes were as large as several to several tens of micrometers. The microscopic Raman spectrum of this sample was measured. The measurement was made on a portion (area C) including the nucleus, a craze portion (area B) excluding the nucleus and a portion (area A) excluding the nucleus and craze. The obtained spectra are shown in Fig. 2. C in Fig. 2 shows the spectrum of the area (area C) having a diameter of several micrometers and including the nucleus, B shows the spectrum of the craze portion (area B) excluding the nucleus, and A shows the spectrum of the portion (area A) excluding the nucleus and craze. D shows the spectrum of the difference between C and A. ○ in Figure shows an absorption based on an aromatic ring.

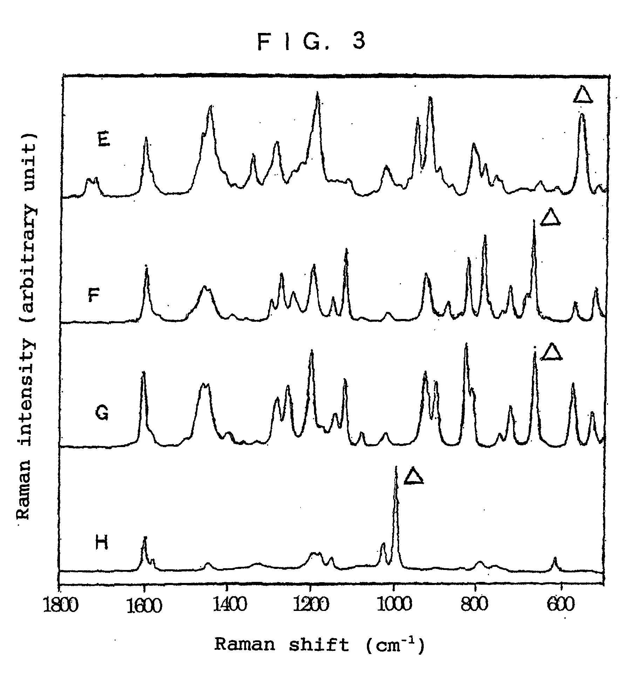

As obvious from Fig. 2, the area A shows the spectrum of typical hydrogenated styrene. A weak peak derived from an aromatic group was also observed at 1,600 cm⁻¹. The area B showed almost the same spectrum as the area A. In contrast to this, in the area C, a strong peak derived from the aromatic group which was observed in the area B and the area A was observed at 1,600 cm⁻¹. This indicates that an aromatic group derived material was concentrated in the area C. To define this peak more clearly, the spectrum (D) of the difference between the area C and the area A was obtained. This spectrum was compared with the standard spectra (Fig. 3) of the Irganox 1010(E), Irgafos 168 (F), 2,4-di-tert-butylphenol (G) which is a hydrolyzate of the Irgafos 168 and polystyrene. In Fig. 3, E denotes the Raman spectrum of the Irganox 1010, F denotes the Raman spectrum of the Irgafos 168, G denotes the Raman spectrum of 2,4-di-tert-butylphenol and H denotes the Raman spectrum of polystyrene. Δ shows a strong absorption not observed in the differential spectrum (D) in Fig. 2. The peak at 1,600 cm⁻¹ was observed in the spectra of the Irganox 1010, Irgafos 168 and 2,4-di-tert-butylphenol but a strong peak at around 600 cm⁻¹ observed in the spectrum of the Irganox 1010, a strong peak at around 650 cm⁻¹ observed in the spectrum of the Irgafos 168 and a strong peak at around 650 cm⁻¹ observed in the spectrum of 2,4-di-tert-butylphenol were not observed in the differential spectrum. Therefore, it can be concluded that the differential spectrum was not based on the Irganox 1010 itself as well as the Irgafos 168 and a decomposed product thereof. Two peaks were observed at around 1,600 cm⁻¹ in the spectrum of polystyrene but it is judged that this differential spectrum was not derived from polystyrene itself because the shapes of the peaks differed from the shape of the peak in the differential spectrum and a strong peak at a wavelength slightly lower than 1,000 cm⁻¹ observed in the spectrum of polystyrene was not observed in the differential spectrum.

When the above results are taken into account, it can be concluded that the differential spectrum is based on a decomposed product of polystyrene hydride comprising an unhydrogenated styrene unit and a decomposed product derived from the Irganox 1010 and/or the Irgafos 168.

### Comparative Example 2

A hydrogenated styrene polymer was obtained by distilling off the solvent without adding a stabilizer to the achromatic transparent solution after filtration obtained in Example 1. The hydrogenation rate determined by ¹H-NMR of the polymer was 99.3 % or more. The reduced viscosity (ηsp/c) measured in a toluene solution having a concentration of 0.5 g/dL at 30°C of the polymer was 0.44 dL/g.

The hydrogenated styrene polymer was ground to obtain granules for molding, and 3 batches of the obtained granules were collected to mold a disk substrate using a CD stamper. Injection molding was carried out at a resin temperature of 330° C and a mold temperature of 120° C but the disk substrate greatly distorted and cracked in most cases.

### Comparative Example 3

3.1 g (0.4 wt% based on the hydrogenated styrene polymer) of the Adecastab AO 330 (of Asahi Denka Kogyo K.K.) was added to the achromatic transparent solution after filtration obtained in Example 1 and then the solvent was distilled off to obtain a hydrogenated styrene polymer. The hydrogenation rate determined by ¹H-NMR of the polymer was 99.3 %. The reduced viscosity (ηsp/c) measured in a toluene solution having a concentration of 0.5 g/dL at 30° C of the polymer was 0.45 dL/g.

The hydrogenated styrene polymer was ground to obtain granules for molding and 3 batches of the obtained granules were collected to mold a disk substrate using a CD stamper. An achromatic transparent disk substrate was injection molded at a resin temperature of 330°C and a mold temperature of 120° C. After the disk substrate was exposed to an atmosphere of 80° C and 85 RH% for 500 hours to promote its deterioration, the number of crazes was counted by observing through a microscope. As a result, the number of crazes was 43 per 1 cm².

### Reference Example 3

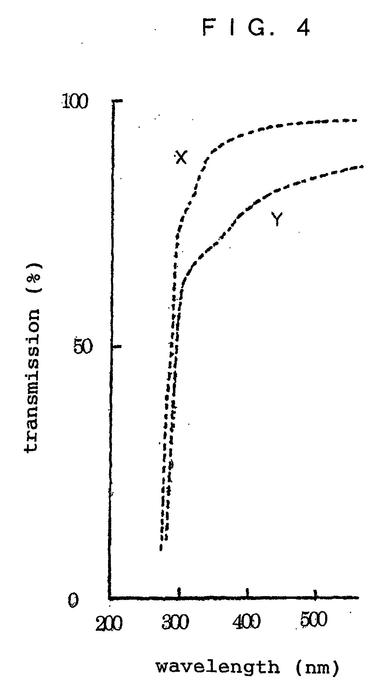

Two samples were obtained from the achromatic transparent solution after filtration obtained in Example 1. One of the samples was directly kept at 260° C or less and flushed under reduced pressure for 4 hours (sample X). The other sample was mixed with 0.4 % of the Adecastab AO 330 (of Asahi Denka Kogyo K.K.) based on a polymer and flushed under the same conditions as the sample X (sample Y). 20 % cyclohexane solutions of the samples X and Y were prepared. The sample X solution was slightly cloudy while the sample Y solution was markedly cloudy. The UV-VIS spectra of these solutions were measured for determination. The results are shown in Fig. 4. As obvious from Fig. 4, the transmission at a visible range of the sample Y was much lower than that of the sample X. That is, it was found that when the sample was flushed at a high temperature for a long time, it became cloudy and the cloudiness was higher when the Adecastab AO 330 (of Asahi Denka Kogyo K.K.) was added than when not added.

### Example 2

3.1 g (0.4 wt% based on the hydrogenated styrene polymer) of the Sumilizer GM (of Sumitomo Chemical Co., Ltd.) (in the above formula (1'), R¹ = tert-butyl group, R² = methyl group, R³ = R⁴ = hydrogen atom) was added to the achromatic transparent solution after filtration obtained in Example 1 and the solvent was distilled off to obtain a hydrogenated styrene polymer. The hydrogenation rate determined by ¹H-NMR of this polymer was 99.3 %. The reduced viscosity (ηsp/c) measured in a toluene solution having a concentration of 0.5 g/dL at 30° C of the polymer was 0.46 dL/g.

The hydrogenated styrene polymer was ground to obtain granules for molding and 5 batches of the obtained granules were collected to injection mold an achromatic transparent disk substrate at a resin temperature of 330° C and a mold temperature of 120° C using a CD stamper. The disk substrate had an extremely low water absorption coefficient of 0.01 % or less and extremely high transparency with a total transmission of 91 % and a haze value of 1.3 %. The birefringence index of the peripheral portion of the obtained disk substrate was extremely low at 8 nm. In other words, the disk substrate showed high optical isotropy.

After the disk substrate was exposed to an atmosphere of 80° C and 85 RH% for 500 hours to promote its deterioration, the number of crazes was counted by observing through a microscope. As a result, the number of crazes on the surface of the substrate was 4 per 1 cm².

### Example 3

After the inside of a 10-liter stainless steel autoclave equipped with agitating elements was fully dried and substituted with nitrogen, 3,360 g of cyclohexane and 288 g of styrene were fed to the autoclave. 4.0 mmols of sec-butyl lithium was then added in the form of a 1.0 M cyclohexane solution to start polymerization. After the resulting solution was stirred at 45° C for 2 hours to completely react styrene, 65 g of isoprene was added to further carry out the reaction at 50° C for 2 hours. Then, 280 g of styrene was added to continue the reaction at 50° C for 2 hours. To 1,950 g of this copolymer solution were added 50 g of an Ni/silica·alumina catalyst (amount of Ni carried: 65 wt%), 530 g of methyl-tert-butyl ether and 1,500 g of cyclohexane in order to carry out a hydrogenation reaction at a hydrogen pressure of 100 kg/cm² and a temperature of 180° C for 6 hours. After the temperature was returned to normal temperature and the inside of the autoclave was fully substituted with nitrogen, the solution was taken out from the autoclave and filtered with a membrane filter having an opening diameter of 0.1 µm (Fluoropore of Sumitomo Electric Industries, Ltd.) under pressure to obtain an achromatic transparent solution.

1.2 g (0.4 wt% based on the hydrogenated styrene-isoprene copolymer) of the Sumilizer GS (of Sumitomo Chemical Co., Ltd.) was added as a stabilizer to this solution and the solvent was distilled off by vacuum concentration and flushing to obtain an achromatic transparent linear hydrogenated styrene-isoprene-styrene bulk tri-block copolymer. The reduced viscosity (ηsp/c) measured in a toluene solution having a concentration of 0.5 g/dL at 30°C of the copolymer was 0.47 dL/g. The hydrogenation rate determined by ¹H-NMR of the copolymer was 99.3 %. It was found by ICP emission spectral analysis that the residual metals were contained in the resin in an amount of 1 ppm or less each, that is, the content of Ni was 0.25 ppm, the content of Al was 0.15 ppm, and the content of Si was 0.13 ppm. The glass transition temperature (Tg) measured by DSC of the copolymer was 147° C.

Thereafter, 5 batches of the obtained granules were collected to injection mold an achromatic transparent disk substrate at a resin temperature of 300° C and a mold temperature of 100° C using a CD stamper. This disk substrate had an extremely low water absorption coefficient of 0.01 wt% or less and extremely high transparency with a total transmission of 91 % and a haze value of 1.3 %. The birefringence index of the peripheral portion of the obtained disk substrate was extremely low at 8 nm. In other words, the disk substrate showed high optical isotropy.

After the disk substrate was exposed to an atmosphere of 80° C and 85 RH% for 500 hours to promote its deterioration, the number of crazes was counted by observing through a microscope. As a result, the number of crazes on the surface of the substrate was 2 per 1 cm².

### Example 4

After the inside of a 10-liter stainless steel autoclave equipped with agitating elements was fully dried and substituted with nitrogen, 2,400 g of cyclohexane, 1,600 g of methyl-tert-butyl ether and 720 g of styrene were fed to the autoclave. 16.2 mmols of n-butyl lithium was then added in the form of a 1.57 M cyclohexane solution to start polymerization. After the resulting solution was stirred at 30° C for 1.5 hours to completely react styrene, 80 g of isoprene was added to further carry out the reaction at 30°C for 1.5 hours. Then, 0.54 g of tetramethoxysilane was added in the form of a 3.0 wt% cyclohexane solution and the temperature was elevated to 60° C to carry out a coupling reaction. 3 hours after the start of the coupling reaction, 10 ml of ethanol was added to the reaction solution. To this copolymer solution was added 140 g of an Ni/silica·alumina catalyst (amount of Ni carried: 65 wt%) in order to carry out a hydrogenation reaction at a hydrogen pressure of 100 kg/cm² and a temperature of 180° C for 6 hours. After the temperature was returned to normal temperature and the inside of the autoclave was fully substituted with nitrogen, the solution was taken out from the autoclave and filtered with a membrane filter having an opening diameter of 0.1 µm (Fluoropore of Sumitomo Electric Industries, Ltd.) under pressure to obtain an achromatic transparent solution. 3.0 g (0.4 wt% based on the hydrogenated styrene-isoprene copolymer) of the Sumilizer GS (of Sumitomo Chemical Co., Ltd.) was added as a stabilizer to this solution and the solvent was distilled off by vacuum concentration and flushing to obtain an achromatic transparent hydrogenated styrene-isoprene bulk copolymer.

The reduced viscosity (ηsp/c) measured in a toluene solution having a concentration of 0.5 g/dL at 30° C of the polymer was 0.48 dL/g. When the polymer was measured by GPC, it was found that the polymer was a mixture of 83 % of a component having four branched chains and 17 % of a component having a single chain, that is, one uncoupled branched chain and a number average molecular weight of 48,000. The hydrogenation rate determined by ¹H-NMR of the polymer was 99.3 %. It was found by ICP emission spectral analysis that the residual metals were contained in the resin in an amount of 1 ppm or less each, that is, the content of Ni was 0.23 ppm, the content of Al was 0.22 ppm, and the content of Si was 0.15 ppm. The melt viscosity measured at 300°C of the polymer was 2,400 poise at a shear rate of 10²s⁻¹ and 850 poise at a shear rate of 10³s⁻¹. The glass transition temperature (Tg) measured by DSC of the polymer was 148° C.

5 batches of the thus obtained granules were collected to injection mold an achromatic transparent disk substrate at a resin temperature of 300° C and a mold temperature of 100° C using a CD stamper. This disk substrate had an extremely low water absorption coefficient of 0.01 wt% or less and extremely high transparency with a total transmission of 91 % and a haze value of 1.6 %. The birefringence index of the peripheral portion of the obtained disk substrate was extremely low at 4 nm. In other words, the disk substrate showed high optical isotropy.

After the disk substrate was exposed to an atmosphere of 80° C and 85 RH% for 500 hours to promote its deterioration, the number of crazes was counted by observing through a microscope. As a result, the number of crazes on the surface of the substrate was 0 per 1 cm².

### Comparative Example 4

A DVD disk substrate was molded in the same manner as in Example 1 except that 3.1 g of IRGANOX HP2225FF (15 % of HP136, 42.5 % of IRGANOX 1010, 42.5 % of IRGAFOS168) was used in place of 3.1 g of the Sumilizer GS.

This disk substrate had a transmission at 400 nm of 90 %. When the transmission at 400 nm of this disk substrate was measured again after it was exposed to 60 mW/cm² of ultraviolet radiation for 2 seconds, it was 73 % which means that this disk was markedly colored.

### Example 5

After the inside of a 10-liter stainless steel autoclave equipped with agitating elements was fully dried and substituted with nitrogen, 750 g of polystyrene (Mw = 2.8 x 10⁵), 118 g of an Ni/silica·alumina catalyst, 2,200 g of cyclohexane and 1,500 g of methyl-tert-butyl ether were fed to the autoclave. After the inside of the autoclave was fully substituted with hydrogen, a hydrogen pressure of 100 kgf/cm² (9.8 MPa) was applied to carry out a hydrogenation reaction at 180°C for 6 hours under agitation. After the end of the reaction, the obtained suspension (slurry) was filtered with a membrane filter having an opening diameter of 0.1 µm (Fluoropore of Sumitomo Electric Industries, Ltd.) under pressure to obtain an achromatic transparent hydrogenated styrene polymer solution.

The Sumilizer GS as a stabilizer and trimethylolpropane triacrylate as a chain extender were added to this filtrate in amounts of 0.4 wt% and 0.5 wt% based on the hydrogenated styrene polymer, respectively, and then the solvent was distilled off under vacuum (flushed) at 200° C or less to obtain a hydrogenated styrene polymer composition (resin).

The hydrogenation rate determined by ¹H-NMR of this polymer was 99.3 %. The reduced viscosity (ηsp/c) measured in a toluene solution having a concentration of 0.5 g/dL at 30° C of the polymer was 0.48 dL/g. It was found by ICP emission spectral analysis that the residual metals were contained in the resin in an amount of 1 ppm or less each, that is, the content of Ni was 0.18 ppm, the content of Al was 0.28 ppm, and the content of Si was 0.23 ppm. The glass transition temperature measured by DSC of the polymer was 149°C.

The thermal stability of the obtained hydrogenated styrene polymer composition (resin) was evaluated using a Koka type flow tester. The melt viscosity of the resin maintained at 340° C for 5 minutes was 430 poise. As the reduced viscosity of the extruded resin was 0.39 dL/g, a viscosity reduction was small.

The hydrogenated styrene polymer composition (resin) was ground to obtain a granular product for molding. An achromatic transparent disk substrate was injection molded from the obtained granular product at a resin temperature of 330° C and a mold temperature of 120° C using a CD stamper. The obtained disk substrate had a reduced viscosity ηsp/c measured in a toluene solution having a concentration of 0.5 g/dL at 30° C of 0.43 dL/g. Thus, a viscosity reduction was very small. As the disk substrate had a glass transition temperature of 149°C, a reduction in glass transition temperature caused by a reduction in molecular weight was not observed. This disk substrate had an extremely low water absorption coefficient of 0.01 % or less and extremely high transparency with a total transmission of 91 % and a haze value of 1.1 %. The birefringence index of the peripheral portion of the obtained disk substrate was extremely low at 5 nm. In other words, the disk substrate showed high optical isotropy.

After the disk substrate was exposed to an atmosphere of 80° C and 85 RH% for 500 hours to promote its deterioration, the number of crazes was counted by observing through a microscope. As a result, the number of crazes on the surface of the substrate was 1 per 1 cm².

### Comparative Example 5

A hydrogenated styrene polymer was obtained by distilling off the solvent under vacuum at 200° C or less without adding a stabilizer to the achromatic transparent solution after filtration obtained in Example 5. The reduced viscosity ηsp/c measured in a toluene solution having a concentration of 0.5 g/dL at 30° C of the polymer was 0.46 dL/g. The reason why it was lower than the reduced viscosity of the hydrogenated styrene polymer of Example 5 which was 0.48 dL/g is considered to be based on a reduction in molecular weight in the flushing step.

The thermal stability of the hydrogenated styrene polymer was evaluated using a Koka type flow tester. The melt viscosity of the resin maintained at 340° C for 5 minutes was extremely low at 180 poise. The reduced viscosity of the extruded resin was extremely low at 0.22 dL/g. That is, a great reduction in the molecular weight of the resin caused by thermal decomposition during the evaluation of thermal stability was observed.

The hydrogenated styrene polymer was ground to obtain granules for molding. A disk substrate was molded from the obtained granules using a CD stamper. Although injection molding was carried out at the same resin temperature of 330° C and the same mold temperature of 120° C as in Example 5, the distortion of the disk substrate was large and the peripheral portion of the disk substrate cracked in most cases. The reduced viscosity ηsp/c measured at 30° C of the obtained molded disk substrate was 0.29 dL/g. That is, it is considered that the distortion and cracking of the disk substrate were caused by a great reduction in molecular weight in the molding step.

### Comparative Example 6

The Irganox 1010 which is a typical hindered phenol was added to the achromatic transparent solution after filtration obtained in Example 5 in an amount of 0.4 wt% based on the hydrogenated styrene polymer and then the solvent was distilled off under vacuum at 200° C or less to obtain a hydrogenated styrene polymer. The reduced viscosity ηsp/c measured in a toluene solution having a concentration of 0.5 g/dL at 30°C of the polymer was 0.46 dL/g.

The thermal stability of the hydrogenated styrene polymer composition (resin) was evaluated using a Koka type flow tester. The melt viscosity of the resin maintained at 340° C for 5 minutes was extremely low at 200 poise. The reduced viscosity of the extruded resin was extremely low at 0.25 dL/g. That is, a great reduction in the molecular weight of the polymer caused by thermal decomposition during the evaluation of thermal stability was observed.

The hydrogenated styrene polymer composition (resin) was ground to obtain granules for molding. Although an achromatic transparent disk substrate was injection molded from the obtained granules at a resin temperature of 330° C and a mold temperature of 120° C using a CD stamper, the distortion of the disk substrate was large and the peripheral portion of the disk substrate cracked in most cases. An achromatic transparent disk substrate was molded at a resin temperature of 330° C and a mold temperature of 120°C. The reduced viscosity η sp/c measured at 30° C of the obtained molded disk substrate was 0.30 dL/g. That is, it is considered that the distortion and cracking of the disk substrate were caused by a great reduction in molecular weight in the molding step.

### Comparative Example 7

The Sumilizer GS was added as a stabilizer to the achromatic transparent solution after filtration obtained in Example 5 in an amount of 0.4 wt% based on the hydrogenated. styrene polymer and then the solvent was distilled off under vacuum at 200° C or less to obtain a hydrogenated styrene polymer composition. The reduced viscosity ηsp/c measured in a toluene solution having a concentration of 0.5 g/dL at 30° C of the polymer was 0.47 dL/g.

The thermal stability of the hydrogenated styrene polymer composition (resin) was evaluated using a Koka type flow tester. The melt viscosity of the resin maintained at 340° C for 5 minutes was 380 poise. The reduced viscosity of the extruded resin was 0.35 dL/g.

### Comparative Example 8

The Irganox HP2225FF was added to the achromatic transparent solution after filtration obtained in Example 5 in an amount of 0.4 wt% based on the hydrogenated styrene polymer and then the solvent was distilled off under vacuum at 200°C or less to obtain a hydrogenated styrene polymer. The reduced viscosity ηsp/c measured in a toluene solution having a concentration of 0.5 g/dL at 30°C of the polymer was 0.47 dL/g.

The thermal stability of the hydrogenated styrene polymer composition (resin) was evaluated using a Koka type flow tester. The melt viscosity of the resin maintained at 340° C for 5 minutes was 360 poise. The reduced viscosity of the extruded resin was 0.35 dL/g.

### Example 6

The Sumilizer GS as a stabilizer and trimethylolpropane triacrylate as a chain extender were added to the achromatic transparent solution after filtration obtained in Example 5 in amounts of 0.4 wt% and 3 wt% based on the hydrogenated styrene polymer, respectively, and then the solvent was distilled off under vacuum (flushed) at 200° C or less to obtain a hydrogenated styrene polymer composition (resin). The reduced viscosity ηsp/c measured in a toluene solution having a concentration of 0.5 g/dL at 30° C of the polymer was 0.48 dL/g. The glass transition temperature measured by DSC of the polymer was 149°C.

The thermal stability of the obtained resin was evaluated using a Koka type flow tester. The melt viscosity of the resin maintained at 340° C for 5 minutes was 520 poise. As the reduced viscosity of the extruded resin was 0.41 dL/g, a reduction in viscosity was small.

The hydrogenated styrene polymer composition (resin) was ground to obtain granules for molding. The obtained granules was injection molded into a disk substrate using a CD stamper. An achromatic transparent disk substrate was molded at a resin temperature of 330° C and a mold temperature of 120° C. The reduced viscosity ηsp/c measured in a toluene solution having a concentration of 0.5 g/dL at 30° C of the obtained disk substrate was 0.44 dL/g. Thus, a viscosity reduction was small. As the glass transition temperature of the disk substrate was 149° C, a reduction in glass transition temperature caused by a reduction in molecular weight was not observed. This disk substrate had an extremely low water absorption coefficient of 0.01 % or less and extremely high transparency with a total transmission of 91 % and a haze value of 1.3 %. The birefringence index of the peripheral portion of the obtained disk substrate was extremely low at 8 nm. In other words, it showed high optical isotropy.

After the disk substrate was exposed to an atmosphere of 80° C and 85 RH% for 500 hours to promote its deterioration, the number of crazes was counted by observing through a microscope. As a result, the number of crazes on the surface of the substrate was 2 per 1 cm².

### Example 7

The Sumilizer GS as a stabilizer and dipentaerythritol hexaacrylate as a chain extender were added to the achromatic transparent solution after filtration obtained in Example 5 in an amount of 0.4 wt% each based on the hydrogenated styrene polymer and then the solvent was distilled off under vacuum (flushed) at 200° C or less to obtain a hydrogenated styrene polymer composition (resin). The reduced viscosity ηsp/c measured in a toluene solution having a concentration of 0.5 g/dL at 30° C of the polymer was 0.48 dL/g.

The thermal stability of the obtained resin was evaluated using a Koka type flow tester. The melt viscosity of the resin maintained at 340° C for 5 minutes was 520 poise. As the reduced viscosity of the extruded resin was 0.40 dL/g, a viscosity reduction was small.

### Example 8

After the inside of a 10-liter stainless steel autoclave equipped with agitating elements was fully dried and substituted with nitrogen, 3,360 g of cyclohexane and 288 g of styrene were fed to the autoclave. 4.0 mmols of sec-butyl lithium was added in the form of a 1.0 M cyclohexane solution to start polymerization. After the resulting solution was stirred at 45° C for 2 hours to completely react styrene, 65 g of isoprene was added to further carry out the reaction at 50° C for 2 hours. 280 g of styrene was then added to continue the reaction at 50° C for 2 hours.

To 1,950 g of this copolymer solution were added 50 g of an Ni/silica·alumina catalyst (amount of Ni carried: 65 wt%) and 530 g of methyl-tert-butyl ether in order to carry out a hydrogenation reaction at a hydrogen pressure of 100 kg/cm² (9.8 MPa) and a temperature of 180° C for 6 hours. After the temperature was returned to normal temperature and the inside of the autoclave was fully substituted with nitrogen, the solution was taken out from the autoclave and filtered with a membrane filter having an opening diameter of 0.1 µm (Fluoropore of Sumitomo Electric Industries, Ltd.) under pressure to obtain an achromatic transparent solution.

The Sumilizer GS as a stabilizer and dimethacrylate of a 1:2 adduct of bisphenol A and ethylene oxide (SR-348) as a chain extender were added to this solution in amounts of 0.4 wt% and 1.0 wt% based on the hydrogenated styrene-isoprene copolymer, respectively, and then the solvent was distilled off by vacuum concentration and flushing to obtain an achromatic transparent linear hydrogenated styrene-isoprene-styrene bulk tri-block copolymer composition (resin).

The reduced viscosity ηsp/c measured in a toluene solution having a concentration of 0.5 g/dL at 30° C of the copolymer was 0.47 dL/g. The hydrogenation rate determined by ¹H-NMR of this copolymer was 99.3 %. It was found by ICP emission spectral analysis that the residual metals were contained in the resin in an amount of 1 ppm or less each, that is, the content of Ni was 0.25 ppm, the content of Al was 0.15 ppm, and the content of Si was 0.13 ppm. The glass transition temperature measured by DSC of the copolymer was 147° C.

The thermal stability of the obtained linear hydrogenated styrene-isoprene-styrene tri-block copolymer composition (resin) was evaluated using a Koka type flow tester. As the reduced viscosity of the resin maintained at 340°C for 5 minutes and extruded was 0.44 dL/g, a reduction in viscosity was extremely small.

An injection molding test was carried out using a granular product obtained by grinding the thus obtained linear hydrogenated styrene-isoprene-styrene tri-block copolymer composition (resin). Molding was carried out at a resin temperature of 300°C and a mold temperature of 100°C. The obtained test sample was solid and transparent. The reduced viscosity ηsp/c measured in a toluene solution having a concentration of 0.5 g/dL at 30° C of this test sample was 0.47 dL/g. This molded product had an extremely low water absorption coefficient of 0.01 % or less and extremely high transparency with a total transmission of 91 % and a haze value of 1.1 %.

### Example 9

After the inside of a 10-liter stainless steel autoclave equipped with agitating elements was fully dried and substituted with nitrogen, 3,380 g of cyclohexane and 553 g of styrene were fed to the autoclave. 9.8 mmols of sec-butyl lithium was added in the form of a cyclohexane solution to start polymerization. After the resulting solution was stirred at 45°C for 2 hours to polymerize styrene, 61 g of isoprene was added to further carry out the reaction at 45° C for 2 hours. To this solution was added 0.65 g of 1,2-bis(trimethoxysilyl)ethane in the form of a 16 wt% cyclohexane solution and the temperature was elevated to 55° C to carry out a coupling reaction. 2 hours after the start of the coupling reaction, 10 ml of ethanol was added. A small amount of the solution was extracted from the obtained polymer solution and injected into a large amount of ethanol, and the precipitated white flaky solid was dried to obtain a styrene-isoprene radial copolymer.

The obtained copolymer had an isoprene content of 9.0 wt% and a reduced viscosity ηsp/c measured in a toluene solution having a concentration of 0.5 g/dL at 30°C of 0.73 dL/g.

To 4,000 g of the above styrene-isoprene radial copolymer solution was added 50 g of an Ni/silica·alumina catalyst (amount of Ni carried: 65 wt%) in order to carry out a hydrogenation reaction at a hydrogen pressure of 100 kg/cm² (9.8 MPa) and a temperature of 180° C for 4 hours in accordance with Example 5. After the temperature was returned to normal temperature and the inside of the autoclave was fully substituted with nitrogen, the solution was taken out from the autoclave and filtered with a membrane filter having an opening diameter of 0.1 µm (Fluoropore of Sumitomo Electric Industries, Ltd.) under pressure to obtain an achromatic transparent solution.

The Sumilizer GM as a stabilizer and dimethacrylate of a 1:2 adduct of bisphenol A and ethylene oxide (SR-348) as a chain extender were added to this solution in an amount of 0.4 wt% each based on the hydrogenated styrene polymer and then the resulting solution was vacuum concentrated (flushed) at 200° C or less to obtain an achromatic hydrogenated styrene-isoprene bulk radial copolymer composition (resin). The reduced viscosity ηsp/c measured in a toluene solution having a concentration of 0.5 g/dL at 30° C of the obtained copolymer was 0.44 dL/g. It was found by ICP emission spectral analysis that the residual metals were contained in the resin in an amount of 1 ppm or less each, that is, the content of Ni was 0.25 ppm, the content of Al was 0.12 ppm, and the content of Si was 0.20 ppm. The glass transition temperature measured by DSC of the copolymer was 147°C.

The thermal stability of the obtained resin was evaluated using a Koka type flow tester. The melt viscosity of the resin maintained at 340° C for 5 minutes was 750 poise. As the reduced viscosity of the extruded resin was 0.42 dL/g, a reduction in viscosity was extremely small.

An injection molding test was carried out using granules obtained by grinding the obtained resin. Molding was carried out at a resin temperature of 300°C and a mold temperature of 100° C. The obtained test sample was solid and transparent. This test sample had a reduced viscosity ηsp/c measured in a toluene solution having a concentration of 0.5 g/dL at 30° C of 0.42 dL/g. This molded product had an extremely low water absorption coefficient of 0.01 % or less and extremely high transparency with a total transmission of 91 % and a haze value of 1.4 %.

### Comparative Example 9

A hydrogenated styrene polymer was obtained by distilling off the solvent at 200° C or less under vacuum without adding a stabilizer to the achromatic transparent solution after filtration obtained in Example 9. The reduced viscosity ηsp/c measured in a toluene solution having a concentration of 0.5 g/dL at 30°C of the polymer was 0.40 dL/g.

The thermal stability of the hydrogenated styrene-isoprene radial copolymer was evaluated using a Koka type flow tester. The melt viscosity of the resin maintained at 340° C for 5 minutes was extremely low at 180 poise. The reduced viscosity of the extruded resin was extremely low at 0.22 dL/g. That is, a great reduction in molecular weight caused by thermal decomposition was observed during the thermal stability test.

### Example 10

343 g of dicyclopentadiene, 1,300 g of toluene and 35 g of triisobutyl aluminum were added to a 3-liter stainless steel reactor. The autoclave was pressurized with 1.5 kgf/cm² (0.147 MPa) of ethylene, and a toluene solution containing 124 mg of isopropylidene-(9-fluorenyl)(cyclopentadienyl)zirconium dichloride and 3 g of triisobutyl aluminum and a toluene solution containing 250 mg of trityl-tetrakis(pentafluorophenyl)borate were added to carry out polymerization at 30° C. During polymerization, 1.5 kgf/cm² (0.147 MPa) of ethylene was always supplied and the supply of ethylene was stopped when 2.3 mols of ethylene was consumed to obtain a polymer solution. The obtained ethylene-dicyclopentadiene copolymer had a Tg of 154°C and a reduced viscosity of 0.78 dL/g, and the molar fraction of dicyclopentadiene in the copolymer was 45 %.

The copolymer solution was transferred to a 5-liter autoclave, and a toluene solution containing 3.0 g of tris(acetylacetonate)cobalt and 4.8 g of triisobutyl aluminum was added to the autoclave. The autoclave was pressurized with 40 kg/cm² (3.92 MPa) of hydrogen to carry out a hydrogenation reaction at 110° C for 3 hours in order to obtain an ethylene hydride-dicyclopentadiene copolymer. The obtained hydride copolymer had a Tg of 153°C, a reduced viscosity of 0.47 dL/g and a hydrogenation rate of 99.9 % or more.

21 g of lactic acid and 2.7 g of water were added to the hydride copolymer solution to carry out a reaction at 95°C for 2 hours so as to precipitate the polymerization catalyst and the hydrogenation catalyst. The solution mixture was filtered with cerite to obtain an ethylene hydride-dicyclopentadiene copolymer solution containing substantially no catalytic residue.

The Sumilizer GS as a stabilizer and dimethacrylate of a 1:2 adduct of bisphenol A and ethylene oxide (SR-348) as a chain extender were added to the obtained copolymer solution in amounts of 0.4 wt% and 1.0 wt% based on the ethylene hydride-dicyclopentadiene copolymer, respectively, and then the solvent was distilled off by vacuum concentration and flushing to obtain an achromatic bulk resin.

When the thermal stability of the obtained ethylene hydride-dicyclopentadiene copolymer composition (resin) was evaluated using a Koka type flow tester, the resin maintained at 340° C for 5 minutes had a reduced viscosity of 0.46 dL/g. Thus, almost no reduction in viscosity was seen.

Then, the obtained resin was injection molded at a resin temperature of 300°C and a mold temperature of 100°C. The obtained test sample was solid and transparent. As the test sample had a reduced viscosity ηsp/c measured in a toluene solution having a concentration of 0.5 g/dL at 30° C of 0.47 dL/g, no reduction in molecular weight was observed. This molded product had an extremely low water absorption coefficient of 0.01 % or less and extremely high transparency with a total transmission of 91 % and a haze value of 1.5 %.

### Example 11

285 g of 8-ethylidene tetracyclo[4.4.0.1^{2,5}.1^{7,10}]-3-dodecene, 1,100 g of toluene, 4.2 g of 1-hexene, 7.5 g of triethylamine and 15 g of triisobutyl aluminum were added to a 3-liter stainless steel reactor and 2.8 g of titanium tetrachloride was further added to carry out polymerization at -10°C for 2 hours so as to obtain a ring-open polymer. A polymer obtained by dispensing a small amount of the obtained solution and purifying it in accordance with a commonly used method had a reduced viscosity ηsp/c measured in a toluene solution having a concentration of 0.5 g/dL at 30° C of 0.65 dL/g and a Tg measured by DSC of 186° C.

7.8 g of lactic acid and 1.0 g of water were added to the obtained reaction solution at 100°C under agitation to carry out a reaction at the same temperature for 2 hours. The black brown reaction solution turned to a black cloudy slurry. The slurry was then filtered. The obtained filtrate was adsorbed to basic alumina to obtain an achromatic solution. The thus obtained solution was added to a large amount of ethanol, and the precipitate was separated by filtration and dried to obtain an achromatic flaky polymer.

The obtained flaky polymer was dissolved in 1,100 g of toluene introduced into an autoclave. The air in the autoclave filled with the solution was fully substituted with nitrogen gas. 3.0 g of tris(acetylacetonate)cobalt and 4.8 g of triisobutyl aluminum were added to the solution to carry out a hydrogenation reaction at a hydrogen pressure of 45 kg/cm² (4.41 MPa) for 120 minutes so as to obtain a reaction solution. A polymer obtained by dispensing a small amount of the reaction solution and purifying it in accordance with a commonly used method had a hydrogenation rate determined by ¹H-NMR of 99.9 % or more. The polymer had a reduced viscosity ηsp/c measured in a toluene solution having a concentration of 0.5 g/dL at 30° C of 0.54 dL/g and a Tg measured by DSC of 140° C.

A lactic acid aqueous solution containing 17.6 g of lactic acid and 1.8 g of water was added to the obtained reaction solution at 100°C under agitation to carry out a reaction at the same temperature for 2 hours. The black brown reaction solution turned to a pink cloudy slurry. The slurry was then filtered. The obtained filtrate was adsorbed to basic alumina to obtain an achromatic solution. The Sumilizer GS as a stabilizer and triallyl trimellitate as a crosslinking agent were added to the obtained solution in an amount of 0.4 wt% each based on the hydrogenated ring-open polymer and then the solvent was distilled off by vacuum concentration and flushing to obtain an achromatic transparent bulk resin. This resin had a reduced viscosity ηsp/c measured in a toluene solution having a concentration of 0.5 g/dL at 30° C of 0.54 dL/g.

The thermal stability of the hydrogenated ring-open polymer (resin) was evaluated using a Koka type flow tester. As the reduced viscosity of the resin maintained at 340°C for 5 minutes was 0.54 dL/g, no reduction in viscosity was seen.

The obtained resin was injection molded at a resin temperature of 340° C and a mold temperature of 100° C. The obtained test sample was solid and transparent. As this test sample had a reduced viscosity ηsp/c measured in a toluene solution having a concentration of 0.5 g/dL at 30° C of 0.54 dL/g, no reduction in molecular weight was observed. This molded product had an extremely low water absorption coefficient of 0.01 % or less and extremely high transparency with a total transmission of 90 % and a haze value of 1.5 %.

### Example 12

(1) After the inside of a 10-liter stainless steel autoclave equipped with agitating elements was fully dried and substituted with nitrogen, 750 g of polystyrene (number average molecular weight Mn = 2.8 x 10⁵), 118 g of an Ni/silica·alumina catalyst, 2,200 g of cyclohexane and 1,500 g of methyl-tert-butyl ether were fed to the autoclave. After the inside of the reactor was fully substituted with hydrogen, a hydrogen pressure of 9.8 MPa was applied to carry out a hydrogenation reaction at 180° C for 6 hours under agitation. After the end of the reaction, the obtained suspension (slurry) was filtered with a membrane filter having an opening diameter of 0.1 µm (Fluoropore of Sumitomo Electric Industries, Ltd.) under pressure to obtain an achromatic transparent solution. A precipitated solid obtained by dispensing part of the solution and injecting it into isopropanol was separated by filtration, cleaned and dried to obtain polystyrene hydride.
   The hydrogenated polystyrene had a hydrogenation rate determined by ¹H-NMR of 99.5 % and a reduced viscosity ηsp/c measured in a toluene solution having a concentration of 0.5 g/dL at 30°C of 0.49 dL/g. It was found by ICP emission spectral analysis that the residual metals were contained in the resin in an amount of 1 ppm or less each, that is, the content of Ni was 0.21 ppm, the content of Al was 0.25 ppm, and the content of Si was 0.25 ppm. The glass transition temperature measured by DSC of the resin was 149°C.
(2) 15 g of resorcin, 70 ml of 95 % ethanol and 34 ml of concentrated hydrochloric acid were fed to a three-necked flask. 7.6 ml of acetaldehyde was added dropwise to this solution at 15° C or less. The obtained solution was heated at 50°C for 1 hour under agitation. When the solution was cooled to room temperature, a white solid precipitated. This precipitate was separated by filtration, washed with water and recrystallized from ethanol-water. In the ¹H-NMR spectrum measured in acetone deuteride of the obtained solid, a peak derived from a hydroxyl group proton was observed at δ8.7 ppm, a peak derived from an aromatic proton was observed at δ7.6 ppm and δ6.2 ppm, a peak derived from a bridging methine proton was observed at δ4.5 ppm and a peak derived from a bridging methyl proton was observed at 61.7 ppm. Thus, it was confirmed that the solid was a cyclic phenol-aldehyde condensate of the above formula (3) in which R⁶ was a hydrogen atom, R⁷ was a methyl group and r was 4. It was also found from the intensity ratio of the peak derived from the aromatic proton to the peak derived from the bridging group that the average number of the residual phenol groups was 8.
   3.0 g of the above cyclic phenol-aldehyde condensate, 2.2 g of triethylamine and 40 ml of acetonitrile were fed to a three-necked flask and mixed together for 20 minutes. The solution was cooled to 0° C and a solution of 2.0 g of acrylic acid chloride dissolved in 20 ml of tetrahydrofuran was added stepwise to the solution in 0.5 hour. After addition, the reaction solution was stirred at normal temperature overnight. Triethylamine hydrochloric acid salt was separated from the obtained mixture by filtration and the solvent was distilled off from the filtrate to obtain a white solid. In the ¹H-NMR spectrum in heavy chloroform of this solid, a peak derived from a hydroxyl group proton was observed at δ5.30 to δ5.60 ppm, a peak derived from an aromatic proton was observed at δ6.82 to δ7.10 ppm, a peak derived from a bridging methine proton was observed at δ4.00 to δ4.80 ppm, a peak derived from a bridging methyl proton was observed at δ1.49 ppm, and a peak derived from an acryl group proton was observed at δ5.75 to δ6.80 ppm, thereby confirming the formation of an acrylated phenol-aldehyde condensate. It was also found from the intensity ratio of the peak derived from the acryl group to the peak derived from the aromatic proton that the acrylation rate was 50 %. In the FD-MS spectrum, a peak at m/z = 760 corresponding to 4 residual phenol groups and an acrylation rate of 50 % was observed. The evaporation start temperature of the acrylated phenol-aldehyde condensate was 376° C which means that the condensate hardly volatilized.
(3) The polyfunctional addition stabilizer obtained in (2) above was added to the achromatic transparent solution of hydrogenated styrene obtained in (1) above in an amount of 0.4 part by weight based on 100 parts by weight of the hydrogenated styrene polymer and then the solvent was distilled off to obtain a polyfunctional addition stabilizer composition comprising the hydrogenated styrene polymer. The composition was ground to obtain granules for molding. The obtained granules was molded (injection/compression molded) into a disk substrate using a CD stamper. An achromatic transparent disk substrate was molded at a resin temperature of 330° C and a mold temperature of 120° C. As the obtained disk substrate had a reduced viscosity ηsp/c measured in a toluene solution having a concentration of 0.5 g/dL at 30° C of 0.46 dL/g, a reduction in viscosity was small. Since the disk substrate had a glass transition temperature (Tg) of 149° C, a reduction in glass transition temperature caused by a reduction in molecular weight was not observed. This disk substrate had an extremely low water absorption coefficient of 0.01 % or less and extremely high transparency with a total transmission of 91 % and a haze value of 1.4 %. The birefringence index of the peripheral portion of the obtained disk substrate was extremely low at 6 nm. In other words, the disk substrate showed extremely high optical isotropy. After the disk substrate was exposed to an atmosphere of 80°C and 85 RH% for 500 hours to promote its deterioration, the number of crazes was counted by observing through a microscope. As a result, the number of crazes on the surface of the substrate was 3 per 1 cm².

The stability of the composition was evaluated using a Koka type flow tester. The reduced viscosity (ηsp/c) of a resin obtained by supplying the resin to a nozzle maintained at 340°C, compressing, keeping it warm for 5 minutes and extruding it from the nozzle was measured. As ηsp/c was 0.44 dL/g, a reduction in viscosity was small.

### Comparative Example 10

A hydrogenated styrene polymer was obtained by distilling off the solvent without adding a polyfunctional addition stabilizer to the achromatic transparent solution of hydrogenated styrene obtained in (1) of Example 12. The polymer was ground to obtain granules for molding. The obtained granules was molded under the same conditions as in Example 12. As a result, it was found that the disk substrate greatly distorted and cracked in most cases. The obtained disk substrate had a ηsp/c of 0.39 dL/g. That is, it is considered that a large reduction in molecular weight occurred in the molding step. The stability of the disk substrate was evaluated using the Koka type flow tester shown in Example 12. As ηsp/c of the disk substrate was 0.27 dL/g, an extremely large reduction in viscosity was observed.

The structure of a decomposed product of the resin was assigned by thermal decomposition GC-MS. Thermal decomposition was carried out at 300° C for 10 minutes in a nitrogen atmosphere. As a result, peaks were observed at m/z = 84 at a hold time of 3.83 minutes, at m/z = 136 at a hold time of 14.92 minutes, at m/z = 208 at a hold time of 22.15 minutes, m/z = 222 at a hold time of 23.05 minutes, m/z = 326 at a hold time of 24.33 minutes, m/z = 334 at a hold time of 25.15 minutes and m/z = 332 at a hold time of 36.60 minutes. It was made clear that this decomposed product was a product in which 1 to 3 units of cyclohexane and vinyl cyclohexane were bonded.

### Comparative Example 11

The Irganox 1010 which is a typical hindered phenolic stabilizer was added to the achromatic transparent solution of hydrogenated styrene obtained in (1) of Example 12 in an amount of 0.4 part by weight based on 100 parts by weight of the hydrogenated styrene polymer and then the solvent was distilled off to obtain a hydrogenated styrene polymer. The heat resistance of the polymer was evaluated by the method of evaluating stability using the Koka type flow tester shown in Example 12. As ηsp/c of the polymer was 0.30 dL/g, an extremely large reduction in viscosity was observed.

### Comparative Example 12

A 1:1 mixture of the Irganox 1010 which is a typical hindered phenolic stabilizer and the Irgafos 168 which is a typical phosphorus-containing stabilizer was added to the achromatic transparent solution of hydrogenated styrene obtained in (1) of Example 12 in an amount of 0.4 part by weight based on 100 parts by weight of the hydrogenated styrene polymer and then the solvent was distilled off to obtain a hydrogenated styrene polymer. The heat resistance of the polymer was evaluated by the method of evaluating stability using the Koka type flow tester shown in Example 12. As ηsp/c of the polymer was 0.37 dL/g, an extremely large reduction in viscosity was observed.

### Example 13

The amount of the polyfunctional addition stabilizer was changed to 0.2 % based on hydrogenated styrene in Example 12 and the stability of the obtained polymer was evaluated using the same Koka type flow tester as in Example 12. As ηsp/c of the resin maintained at 340° C for 5 minutes was 0.42 dL/g, a reduction in viscosity was small.

### Example 14

The amount of the polyfunctional addition stabilizer was changed to 1.0 part by weight based on 100 parts by weight of the hydrogenated styrene polymer in Example 12 and the stability of the obtained polymer was evaluated using the same Koka type flow tester as in Example 12. As ηsp/c of the resin maintained at 340°C for 5 minutes was 0.45 dL/g, a reduction in viscosity was small.

### Example 15

(1) After the inside of a 10-liter stainless steel autoclave equipped with agitating elements was fully dried and substituted with nitrogen, 3,360 g of cyclohexane and 288 g of styrene were fed to the autoclave. 4.0 mmols of sec-butyl lithium was added in the form of a 1.0 M cyclohexane solution to start polymerization. After the resulting solution was stirred at 45° C for 2 hours to completely react styrene, 65 g of isoprene was added to further carry out the reaction at 50°C for 2 hours and then 280 g of styrene was further added to continue the reaction at 50°C for 2 hours. Then, isopropanol was added to terminate the polymerization. 50 g of an Ni/silica·alumina catalyst (amount of Ni carried: 65 wt%) and 530 g of methyl-tert-butyl ether were added to 1,950 g of this copolymer solution to carry out a hydrogenation reaction at a hydrogen pressure of 9.8 MPa and a temperature of 180° C for 6 hours. After the temperature was returned to normal temperature and the inside of the autoclave was fully substituted with nitrogen, the solution was taken out from the autoclave and filtered with a membrane filter having an opening diameter of 0.1 µm (Fluoropore of Sumitomo Electric Industries, Ltd.) under pressure to obtain an achromatic transparent solution of a hydrogenated styrene-isoprene-styrene tri-block copolymer. A precipitated solid obtained by dispensing part of this solution and injecting it into a large amount of isopropanol was separated by filtration, cleaned and dried to obtain a flaky solid of the hydride.
   This solid had a reduced viscosity ηsp/c measured in a toluene solution having a concentration of 0.5 g/dL at 30° C of 0.47 dL/g and a hydrogenation rate determined by ¹H-NMR of 99.5 %. It was found by ICP emission spectral analysis that the residual metals were contained in the resin in an amount of 1 ppm or less each, that is, the content of Ni was 0.28 ppm, the content of Al was 0.15 ppm, and the content of Si was 0.22 ppm. The glass transition temperature (Tg) measured by DSC of the resin was 147°C.
(2) The polyfunctional addition stabilizer obtained in (2) of Example 12 was added to the achromatic solution of the hydrogenated styrene-isoprene-styrene copolymer obtained in (1) above in an amount of 0.4 part by weight based on 100 parts by weight of the copolymer and then the solvent was distilled off to obtain a polyfunctional addition stabilizer composition comprising the hydrogenated styrene polymer. This composition was ground to obtain granules for molding. The obtained granules was molded into a disk substrate using a CD stamper. The achromatic transparent disk substrate was molded at a resin temperature of 330° C and a mold temperature of 120°C. As the obtained disk substrate had a reduced viscosity ηsp/c measured in a toluene solution having a concentration of 0.5 g/dL at 30° C of 0.45 dL/g, a reduction in viscosity was small. Since the disk substrate had a glass transition temperature (Tg) of 147°C, a reduction in glass transition temperature caused by a reduction in molecular weight was not observed. This disk substrate had an extremely low water absorption coefficient of 0.01 % or less and extremely high transparency with a total transmission of 91.5 % and a haze value of 1.2 %. The birefringence index of the peripheral portion of the obtained disk substrate was extremely low at 8 nm. In other words, it showed extremely high optical isotropy. After the disk substrate was exposed to an atmosphere of 80° C and 85 RH% for 500 hours to promote its deterioration, the number of crazes was counted by observing through a microscope. As a result, the number of crazes on the surface of the substrate was 2 per 1 cm².

The stability of the composition was evaluated using the same Koka type flow tester as in Example 12. As the reduced viscosity (ηsp/c) of the resin maintained at 340° C for 5 minutes was 0.44 dL/g, a reduction in viscosity was small.

### Example 16

(1) After the inside of a 10-liter stainless steel autoclave equipped with agitating elements was fully dried and substituted with nitrogen, 2,400 g of cyclohexane, 1,600 g of methyl-tert-butyl ether and 720 g of styrene were fed to the autoclave. 16.2 mmols of n-butyl lithium was then added in the form of a 1.57 Mn-hexane solution to start polymerization. After the resulting solution was stirred at 30° C for 1.5 hours to completely react styrene, 80 g of isoprene was added to further carry out the reaction at 30° C for 1.5 hours. 0.54 g of tetramethoxysilane was added in the form of a 3.0 wt% cyclohexane solution and the temperature was elevated to 60° C to carry out a coupling reaction. 3 hours after the start of the coupling reaction, 10 ml of ethanol was added. To this copolymer solution was added 140 g of an Ni/silica·alumina catalyst (amount of Ni carried: 65 wt%) in order to carry out a hydrogenation reaction at a hydrogen pressure of 9.8 MPa and a temperature of 180°C for 6 hours. After the temperature was returned to normal temperature and the inside of the autoclave was fully substituted with nitrogen, the solution was taken out from the autoclave and filtered with a membrane filter having an opening diameter of 0.1 µm (Fluoropore of Sumitomo Electric Industries, Ltd.) under pressure to obtain an achromatic transparent solution of a hydrogenated styrene-isoprene radial copolymer. A precipitate obtained by dispensing part of the solution and injecting it into a large amount of isopropanol was separated by filtration, cleaned and dried to obtain a solid polystyrene hydride radial copolymer.
   The solid had a reduced viscosity ηsp/c measured in a toluene solution having a concentration of 0.5 g/dL at 30° C of 0.49 dL/g. When it was measured by GPC, it was found to be a mixture of 83 % of a component having four branched chains and 17 % of a component having a single chain, that is, one uncoupled branched chain and a number average molecular weight (Mn) of 48,000. It had a hydrogenation rate determined by ¹H-NMR of 99.3 %. It was found by ICP emission spectral analysis that the residual metals were contained in the resin in an amount of 1 ppm or less each, that is, the content of Ni was 0.24 ppm, the content of Al was 0.32 ppm, and the content of Si was 0.26 ppm.
(2) 20 g of p-tert-butylphenol, 5.0 g of concentrated hydrochloric acid, 15 ml of ethanol and 5.0 g of formaldehyde (37 % aqueous solution) were fed to a 500-ml three-necked flask and heated under reflux at 70 to 80°C for 3 hours. Thereafter, the solvent was distilled off and the obtained reaction solution was fully washed with water. In the ¹H-NMR spectrum measured in heavy chloroform of the obtained white solid, a peak derived from a hydroxyl group proton was observed at δ8.58 ppm and δ9.49 ppm, a peak derived from an aromatic proton was observed at δ6.92 ppm, a peak derived from a bridging methylene proton was observed at δ3.81 to δ3.86 ppm and a peak derived from a tert-butyl proton was observed at δ1.09 to δ1.17 ppm, thereby confirming the formation of a chain phenol-aldehyde condensate of the above formula (1) in which R¹ and R³ were each a hydrogen atom, R² was a tert-butyl group and p was 7.
   To 2.0 g of the obtained chain phenol-aldehyde condensate was added 0.80 g of triethylamine in order to prepare a homogeneous solution. This solution was cooled to 0°C and 20 ml of a tetrahydrofuran solution containing 0.71 g of acrylic acid chloride was added dropwise to the solution in 30 minutes. The reaction solution was stirred at normal temperature overnight. Triethylamine hydrochloric acid salt was separated from the obtained mixture by filtration and the filtrate was concentrated and dried up to obtain a white solid. In the ¹H-NMR spectrum in heavy chloroform of this solid, a peak derived from a tert-butyl proton was observed at δ1.12 to δ1.28 ppm, a peak derived from a bridging methylene proton was observed at δ3.75 ppm, a peak derived from an acryl proton was observed at δ5.81 ppm, δ6.15 ppm and δ6.41 ppm, and a peak derived from an aromatic proton was observed at δ6.80 to δ7.22 ppm, thereby confirming the formation of an acrylated phenol-aldehyde condensate. It was also confirmed from the intensity ratio of the peak derived from the acryl group to the peak derived from the tert-butyl group that the acrylation rate was 40 %. It was also found from the intensity ratio of the peak derived from the bridging group to the peak derived from the tert-butyl group that the average number of the residual phenol groups was 5. The evaporation start temperature of the acrylated phenol-aldehyde condensate was 410°C which means that the condensate hardly volatilized.
(3) The polyfunctional addition stabilizer obtained in (2) above was added to the achromatic solution of the hydrogenated styrene-isoprene radial copolymer obtained in (1) above in an amount of 0.4 part by weight based on 100 parts by weight of the copolymer and then the solvent was distilled off to obtain a polyfunctional addition stabilizer composition comprising the hydrogenated styrene polymer. The stability of the composition was evaluated using the same Koka type flow tester as in Example 12. As the reduced viscosity ηsp/c of the resin maintained at 340° C for 5 minutes was 0.47 dL/g, a reduction in viscosity was small.

### Example 17

(1) 343 g (2.6 mols) of dicyclopentadiene, 1,300 g of toluene and 35 g of triisobutyl aluminum were added to a 3-liter stainless steel reactor. The autoclave was pressurized with 0.15 MPa of ethylene, and a toluene solution containing 124 mg (0.29 mmol) of isopropylidene-(9-fluorenyl)(cyclopentadienyl)zirconium dichloride was added to carry out polymerization at 30°C. 0.15 MPa of ethylene was always supplied during polymerization and the supply of ethylene was stopped when 2.3 mols of ethylene was consumed to obtain a polymer solution. The obtained ethylene-dicyclopentadiene copolymer had a glass transition temperature (Tg) of 153°C and a reduced viscosity ηsp/c of 0.76 dL/g, and the molar fraction of a dicyclopentadiene unit in the copolymer was 44 %.
   The copolymer was transferred to a 5-liter autoclave and a toluene solution containing 3.0 g (8.4 mmols) of tris(acetylacetonate)cobalt and 4.8 g of triisobutyl aluminum was added. The autoclave was pressurized with 3.9 MPa of hydrogen gas to carry out a hydrogenation reaction at 110° C for 3 hours so as to obtain an ethylene hydride-dicyclopentadiene copolymer. The obtained hydride copolymer had a glass transition temperature (Tg) of 152°C, a reduced viscosity ηsp/c of 0.47 dL/g and a hydrogenation rate of 99.9 % or more.
   21 g of lactic acid and 2.7 g of water were added to the hydride copolymer to carry out a reaction at 95°C for 2 hours and the polymerization catalyst and the hydrogenation catalyst were precipitated. The solution mixture was filtered with cerite to obtain an ethylene hydride-dicyclopentadiene copolymer solution containing substantially no catalyst.
(2) 20 g of p-tert-butylphenol, 15.5 ml of a 37 % formaldehyde aqueous solution and 0.35 g of sodium hydroxide were fed to a 500-ml three-necked flask. The obtained solution was heated at 110 to 120°C for 2 hours under agitation. Water was distilled off while a reaction was carried out. A light yellow solid was precipitated during this step. To this mixture was added 200 ml of diphenyl ether in order to dissolve the solid. This solution was bubbled with nitrogen gas to distill off water and refluxed at 250°C for 2 hours in the end. This reaction solution was cooled to room temperature and 400 ml of ethyl acetate was added to precipitate a solid. This solid was separated by filtration, washed with ethyl acetate and water and then dried. In the ¹H-NMR spectrum measured in heavy chloroform of the obtained solid, a peak derived from a hydroxyl group proton was observed at δ10.40 ppm, a peak derived from an aromatic proton was observed at δ7.08 ppm, a peak derived from a bridging methylene proton was observed at δ4.30 ppm and δ3.55 ppm, and a peak derived from a tert-butyl proton was observed at δ1.26 ppm, thereby confirming the formation of a cyclic phenol-aldehyde condensate of the above formula (2) in which R⁴ was a tert-butyl group, R⁵ was a hydrogen atom and q was 4. It was also confirmed from the intensity ratio of the peak derived from the tert-butyl group to the peak derived from the bridging group that the average number of the residual phenol groups was 4.
   1.56 g of the obtained phenol-aldehyde condensate, 20 ml of a solution containing tetrahydrofuran and dimethylformamide in a weight ratio of 4:1 and 0.48 g of triethylamine were fed to a three-necked flask and mixed together. The reaction solution was cooled to 0°C and a solution of 0.43 g of acrylic acid chloride dissolved in 20 ml of tetrahydrofuran was added dropwise to the solution in 30 minutes. After the end of addition, the reaction mixture was stirred for 6 hours. The precipitated triethylamine hydrochloric acid salt was separated by filtration and the filtrate was partially concentrated. The obtained mixture was injected into water and the precipitated white solid was separated by filtration, washed with water and dried. In the ¹H-NMR spectrum measured in heavy chloroform of this solid, a peak derived from an aromatic proton was observed at δ7.02 ppm and δ6.98 ppm, a peak derived from an acryl proton was observed at δ5.90 ppm to δ6.50 ppm and δ3.70 ppm, a peak derived from a bridging methylene proton was observed at δ3.60 ppm, and a peak derived from a tert-butyl proton was observed at δ1.25 ppm and δ1.10 ppm, thereby confirming the formation of an acrylated phenol-aldehyde condensate. It was confirmed from the intensity ratio of the peak at δ1.25 ppm to the peak at δ1.10 ppm that the acrylation rate was 56 %. A peak at m/z of 756 corresponding to 2 residual phenol groups and an acrylation rate of 56 % was confirmed by FD-MS. The evaporation start temperature of the obtained phenol-acrylated phenol condensate was 439° C which means that the condensate hardly volatilized.
(3) The polyfunctional addition stabilizer obtained in (2) above was added to the ethylene hydride-dicyclopentadiene copolymer solution obtained in (1) above in an amount of 0.4 part by weight based on 100 parts by weight of the copolymer and then the solvent was distilled off by vacuum concentration to obtain an achromatic transparent bulk resin. The resin was injection molded at a resin temperature of 300°C and a mold temperature of 100°C. The obtained test sample was transparent and solid. As this test sample had a reduced viscosity ηsp/c measured in a transparent solution having a concentration of 0.5 g/dL at 30° C of 0.46 dL/g, a reduction in molecular weight caused by molding was rarely observed. This molded product had an extremely low water absorption coefficient of 0.01 % or less and extremely high transparency with a total transmission of 91.2 % and a haze value of 2.1 %.

### Example 18

(1) 285 g of 8-ethylidene tetracyclo[4.4.0.1^{2,5}.1^{7,10}]-3-dodecene, 1,100 g of toluene, 4.2 g of 1-hexene, 7.5 g of triethylamine and 15 g of triisobutyl aluminum were added to a 3-liter stainless steel reactor and 2.8 g of titanium tetrachloride was further added to carry out polymerization at -10° C for 2 hours to obtain a ring-open polymer. A polymer obtained by dispensing a small amount of the obtained solution and purifying it in accordance with a commonly used method had a reduced viscosity ηsp/c measured in a toluene solution having a concentration of 0.5 g/dL at 30°C of 0.65 dL/g and a glass transition temperature (Tg) measured by DSC of 188° C. To the obtained reaction solution were added 7.8 g of lactic acid and 1.0 g of water under agitation at 100° C in order to carry out a reaction at the same temperature for 2 hours. The black brown reaction solution turned to a black cloudy slurry. The slurry was then filtered. The obtained filtrate was adsorbed to basic alumina to obtain an achromatic solution. The thus obtained solution was added to a large amount of ethanol and the obtained precipitate was separated by filtration and dried to obtain an achromatic flake.
   The obtained flaky solid was dissolved in 1,100 g of toluene introduced into an autoclave. The air in the autoclave filled with the obtained solution was fully substituted with nitrogen gas. 3.0 g of tris(acetylacetonate)cobalt and 4.8 g of triisobutyl aluminum were added to the solution to carry out a hydrogenation reaction at a hydrogen pressure of 4.4 MPa for 120 minutes so as to obtain a reaction solution. A polymer obtained by dispensing a small amount of the reaction solution and purifying it in accordance with a commonly used method had a hydrogenation rate determined from its ¹H-NMR spectrum of 99.9 % or more, a reduced viscosity ηsp/c measured in a toluene solution having a concentration of 0.5 g/dL at 30°C of 0.55 dL/g and a glass transition temperature (Tg) measured by DSC of 142° C.
   An aqueous solution of 17.6 g of lactic acid dissolved in 1.8 g of water was added to the obtained reaction solution under agitation at 100°C to carry out a reaction at the same temperature for 2 hours. The black brown reaction solution turned to a pink cloudy slurry. The slurry was then filtered. The obtained filtrate was adsorbed to basic alumina to obtain an achromatic transparent hydrogenated ring-open polymer solution.
(2) 18 g of paraformaldehyde, 55.6 g of p-tert-butylphenol, 0.8 ml of 10N-KOH and 300 ml of xylene were fed to a 500-ml three-necked flask and heated under reflux at 140 to 150°C for 4 hour in a stream of nitrogen. After cooling, the precipitated solid was separated by filtration and washed with toluene, ether, acetone and water sequentially. Then, this solid was recrystallized from chloroform to obtain a purified product. In the ¹H-NMR spectrum measured in heavy chloroform of the obtained solid, a peak derived from a hydroxyl group proton was observed at δ9.6 ppm, a peak derived from an aromatic proton was observed at δ7.08 ppm, a peak derived from a bridging methylene proton was observed at δ4.38 ppm and δ3.5 ppm, and a peak derived from a tert-butyl proton was observed at δ1.25 ppm, thereby confirming the formation of a cyclic phenol-aldehyde condensate of the above formula (2) in which R⁴ was a tert-butyl group, R⁵ was a hydrogen atom and q was 8. It was also found from the intensity ratio of the peak derived from the tert-butyl proton to the peak derived from the bridging methylene proton that the average number of the residual phenol groups was 8.

The obtained phenol-aldehyde condensate was acrylated in the same manner as the phenol-aldehyde condensate shown in Example 3 to obtain a white solid. In the ¹H-NMR spectrum measured in heavy chloroform of this solid, a peak derived from an aromatic proton was observed at δ6.50 ppm to δ7.20 ppm, a peak derived from an acryl proton was observed at δ5.41 ppm to 66.43 ppm, a peak derived from a bridging methylene proton was observed at δ3.22 ppm to δ3.92 ppm and a peak derived from a tert-butyl proton was observed at δ0.81 ppm to δ1.32 ppm, thereby confirming the formation of an acrylated phenol-aldehyde condensate. It was also confirmed from the intensity ratio of the peak derived from the tert-butyl proton to the peak derived from the bridging methylene proton that the acrylation rate was 42 % and that the average number of the residual phenol groups was 5.

The polyfunctional addition stabilizer obtained in (2) above was added to the transparent solution of the hydrogenated ring-open polymer obtained in (1) above in an amount of 0.4 part by weight based on 100 parts by weight of the copolymer and then the solvent was distilled off by vacuum concentration to obtain an achromatic transparent bulk resin. The obtained resin was injection molded at a resin temperature of 300°C and a mold temperature of 100°C. The obtained test sample was transparent and solid. As the test sample had a reduced viscosity ηsp/c measured in a transparent solution having a concentration of 0.5 g/dL at 30° C of 0.54 dL/g, a reduction in molecular weight caused by molding was rarely observed. This molded product had an extremely low water absorption coefficient of 0.01 % or less and extremely high transparency with a total transmission of 91.0 % and a haze value of 2.5 %.

## Claims

1. A thermoplastic resin composition comprising:
(A) a thermoplastic polymer having alicyclic groups, and
(B) 0.01 to 5 wt% based on the above thermoplastic polymer of an addition stabilizer which can undergo addition reaction with a radical formed by the cleavage of the above thermoplastic polymer.

2. The thermoplastic resin composition of claim 1, wherein the thermoplastic polymer (A) is a hydrogenated styrene polymer.

3. The thermoplastic resin composition of claim 1, wherein the thermoplastic polymer (A) is a saturated cyclic polymer containing a polymerization unit derived from a cyclic olefin in the main chain.

4. The thermoplastic resin composition of claim 1, wherein the addition stabilizer (B) is a phenol compound having a (meth)acrylate group.

5. The thermoplastic resin composition of claim 1, wherein the addition stabilizer (B) is a compound obtained by converting 35 to 65 mol% of the hydroxyl groups of a chain phenol-aldehyde condensate represented by the following formula (1) into (meth)acryloyloxy groups: 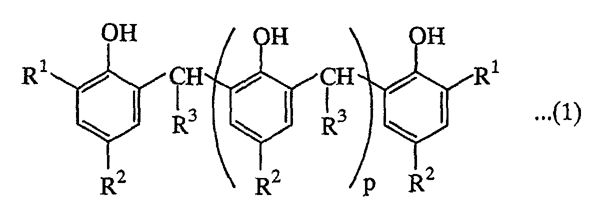 wherein R¹, R² and R³ are each independently a hydrogen atom or alkyl group having 1 to 10 carbon atoms, and p is an integer of 0 to 13, with the proviso that each of R¹'s, R²'s and R³'s may be the same or different.

6. The thermoplastic resin composition of claim 1, wherein the addition stabilizer (B) is a compound represented by the following formula (1'): 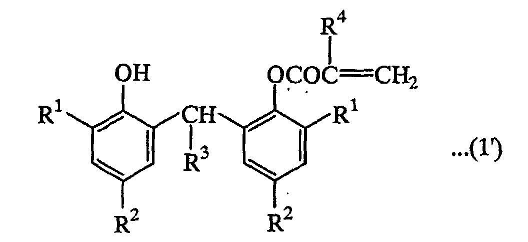 wherein R¹, R² and R³ are as defined in the above formula (1) and R⁴ is a hydrogen atom or methyl group.

7. The thermoplastic resin composition of claim 1, wherein the addition stabilizer (A) is a compound obtained by converting 35 to 65 mol% of the hydroxyl groups of a cyclic phenol-aldehyde condensate represented by the following formula (2) into (meth)acryloyloxy groups: 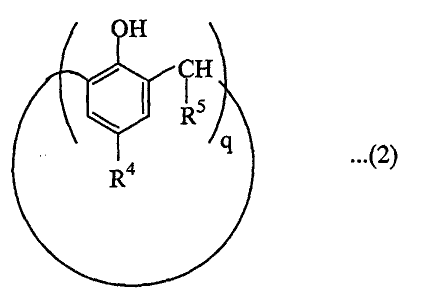 wherein R⁴ and R⁵ are the same or different and each a hydrogen atom or alkyl group having 1 to 10 carbon atoms, and q is an integer of 4 to 15.

8. The thermoplastic resin composition of claim 1, wherein the addition stabilizer (A) is a compound obtained by converting 35 to 65 mol% of the hydroxyl groups of a cyclic phenol-aldehyde condensate represented by the following formula (3) into (meth)acryloyloxy groups: 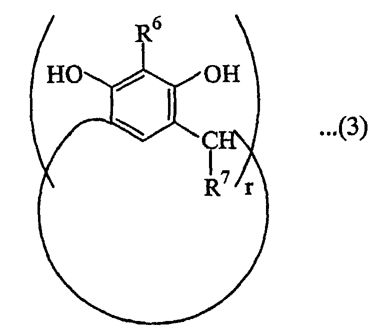 wherein R⁶ and R⁷ are the same or different and each a hydrogen atom or alkyl group having 1 to 10 carbon atoms, and r is an integer of 4 to 15.

9. The thermoplastic resin composition of claim 5, wherein the addition stabilizer (B) is a combination of a compound of the above formula (1) in which p is 0 and a compound of the above formula (1) in which p is 1 to 13.

10. The thermoplastic resin composition of claim 1 which further comprises at least one chain extender selected from the group consisting of a polyfunctional (meth)acrylate compound and polyfunctional (meth)allyl compound of an aliphatic or alicyclic polyol in an amount of 0.05 to 5 wt% based on the thermoplastic polymer (A).

11. The thermoplastic resin composition of claim 1 or 6, wherein when the composition is injection compression molded into a substrate having a diameter of 12 cm and a thickness of 1.2 mm at a resin temperature of 330° C and a mold temperature of 120° C using a metal mold and this substrate is exposed to an atmosphere of 80°C and 85 RH% for 500 hours, only 10 or less crazes are observed per 1 cm² on the surface of the substrate.

12. A molded product obtained by melt molding the thermoplastic resin composition of claim 1.

13. The molded product of claim 12 which is used for optical application.

14. An optical disk substrate which is essentially made from a thermoplastic resin having an alicyclic group and on the surface of the substrate which only 10 or less crazes are observed per 1 cm² when it is exposed to an atmosphere of 80° C and 85 RH% for 500 hours.

15. The optical disk substrate of claim 14 which is made from the thermoplastic resin composition of claim 1.

16. A compound obtained by converting 35 to 65 mol% of the hydroxyl groups of a chain phenol-aldehyde condensate represented by the following formula (1") into (meth)acryloyloxy groups: 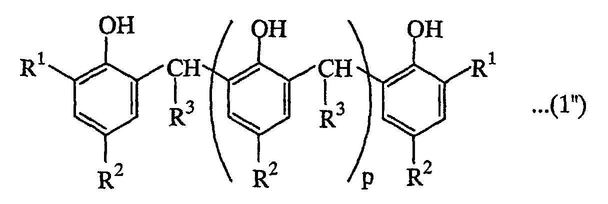 wherein R¹, R² and R³ are each independently a hydrogen atom or alkyl group having 1 to 10 carbon atoms, and p is an integer of 2 to 13, with the proviso that each of R¹'s, R²'s and R³'s may be the same or different.

17. A compound obtained by converting 35 to 65 mol% of the hydroxyl groups of a cyclic phenol-aldehyde condensate represented by the following formula (2) into (meth)acryloyloxy groups: 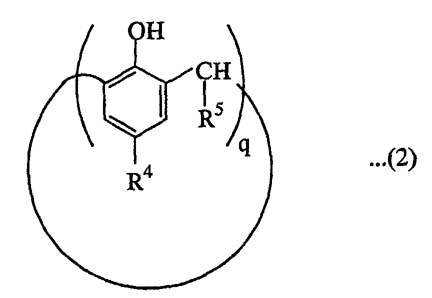 wherein R⁴ and R⁵ are the same or different and each a hydrogen atom or alkyl group having 1 to 10 carbon atoms, and q is an integer of 4 to 15.

18. A compound obtained by converting 35 to 65 mol% of the hydroxyl groups of a cyclic phenol-aldehyde condensate represented by the following formula (3) into (meth)acryloyloxy groups: 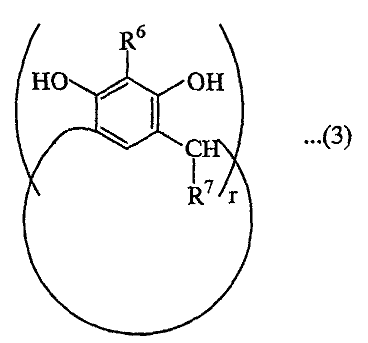 wherein R⁶ and R⁷ are the same or different and each a hydrogen atom or alkyl group having 1 to 10 carbon atoms, and r is an integer of 4 to 15.
